# FASCICULE DE BREVET EUROPEEN

(11) **EP 1 481 065 B1**
(45) Date de publication et mention de la délivrance du brevet: **26.07.2006**
(21) Numéro de dépôt: 03720645.5
(22) Date de dépôt: 25.02.2003
(51) Int. Cl.: C12N 15/62, C07K 14/715, G01N 33/50

(54) **PROCEDE DE DETECTION DE LIGANDS DU RECEPTEUR DE LA LEPTINE**
VERFAHREN ZUM NACHWEIS VON LIGANDEN DES LEPTINREZEPTORS
METHOD FOR DETECTION OF LEPTIN RECEPTOR LIGANDS

(30) Priorité: 26.02.2002 FR 0202431
(43) Date de publication de la demande: 01.12.2004
(73) Titulaire: Aventis Pharma S.A., 92165 Antony Cédex (FR); INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75794 Paris Cedex 16 (FR)
(72) Inventeur: JOCKERS, Ralph, F-91440 BURES SUR YVETTE (FR); COUTURIER, Cyril, F-75014 PARIS (FR)
(74) Mandataire: Bouvet, Philippe
(86) Numéro de dépôt international: PCT/FR2003/000610
(87) Numéro de publication internationale: WO 2003/072787

(56) Documents cités:
- WO-A-97/19552
- WO-A-99/66324
- LUNDIN A ET AL: "Expression and intracellular localization of leptin receptor long isoform-GFP chimera" BIOCHIMICA ET BIOPHYSICA ACTA. MOLECULAR CELL RESEARCH, ELSEVIER SCIENCE PUBLISHERS, AMSTERDAM, NL, vol. 1499, no. 1-2, 11 décembre 2000 (2000-12-11), pages 130-138, XP004278202 ISSN: 0167-4889
- BOUTE N ET AL: "MONITORING THE ACTIVATION STATE OF THE INSULIN RECEPTOR USING BIOLUMINESCENCE RESONANCE ENERGY TRANSFER" MOLECULAR PHARMACOLOGY, BALTIMORE, MD, US, vol. 4, no. 60, octobre 2001 (2001-10), pages 640-645, XP001053695 ISSN: 0026-895X cité dans la demande
- ANGERS S ET AL: "DETECTION OF BETA2-ADRENERGIC RECEPTOR DIMERIZATION IN LIVING CELLS USING BIOLUMINESCENCE RESONANCE ENERGY TRANSFER (BRET)" PROCEEDINGS OF THE NATIONAL ACADEMY OF SCIENCES OF USA, NATIONAL ACADEMY OF SCIENCE. WASHINGTON, US, vol. 97, no. 7, 28 mars 2000 (2000-03-28), pages 3684-3689, XP000986224 ISSN: 0027-8424 cité dans la demande
- COUTURIER C.; JOCKERS R. J.BIOL.CHEM. vol. 278, no. 29, 18 Juillet 2003, pages 26604 - 26611

### LISTE DE SEQUENCES

<110> AVENTIS PHARMA S.A.
   INSERM
<120> Procédé de criblage d'agonistes et d'antagonistes de la leptine
<130> RecepteurLeptineBRET
<140>
   <141>
<160> 8
<170> PatentIn Ver. 2.1
<210> 1
   <211> 2691
   <212> ADN
   <213> Homo sapiens
<220>
   <221> CDS
   <222> (1)..(2691)
<400> 1
   atg att tgt caa aaa ttc tgt gtg gtt ttg tta cat tgg gaa ttt att      48
   Met Ile Cys Gln Lys Phe Cys Val Val Leu Leu His Trp Glu Phe Ile
     1               5                  10                  15
   tat gtg ata act gcg ttt aac ttg tca tat cca att act cct tgg aga      96
   Tyr Val Ile Thr Ala Phe Asn Leu Ser Tyr Pro Ile Thr Pro Trp Arg
                20                  25                  30
   ttt aag ttg tct tgc atg cca cca aat tca acc tat gac tac ttc ctt      144
   Phe Lys Leu Ser Cys Met Pro Pro Asn Ser Thr Tyr Asp Tyr Phe Leu
            35                  40                  45
   ttg cct gct gga ctc tca aag aat act tca aat tcg aat gga cat tat      192
   Leu Pro Ala Gly Leu Ser Lys Asn Thr Ser Asn Ser Asn Gly His Tyr
        50                  55                  60
   gag aca gct gtt gaa cct aag ttt aat tca agt ggt act cac ttt tct      240
   Glu Thr Ala Val Glu Pro Lys Phe Asn Ser Ser Gly Thr His Phe Ser
    65                  70                  75                  80
   aac tta tcc aaa aca act ttc cac tgt tgc ttt cgg agt gag caa gat      288
   Asn Leu Ser Lys Thr Thr Phe His Cys Cys Phe Arg Ser Glu Gln Asp
                    85                  90                  95
   aga aac tgc tcc tta tgt gca gac aac att gaa gga aag aca ttt gtt      336
   Arg Asn Cys Ser Leu Cys Ala Asp Asn Ile Glu Gly Lys Thr Phe Val
               100                 105                 110
   tca aca gta aat tct tta gtt ttt caa caa ata gat gca aac tgg aac      384
   Ser Thr Val Asn Ser Leu Val Phe Gln Gln Ile Asp Ala Asn Trp Asn
           115                 120                 125
   ata cag tgc tgg cta aaa gga gac tta aaa tta ttc atc tgt tat gtg      432
   Ile Gln Cys Trp Leu Lys Gly Asp Leu Lys Leu Phe Ile Cys Tyr Val
       130                 135                 140
   gag tca tta ttt aag aat cta ttc agg aat tat aac tat aag gtc cat      480
   Glu Ser Leu Phe Lys Asn Leu Phe Arg Asn Tyr Asn Tyr Lys Val His
   145                 150                 155                 160
   ctt tta tat gtt ctg cct gaa gtg tta gaa gat tca cct ctg gtt ccc      528
   Leu Leu Tyr Val Leu Pro Glu Val Leu Glu Asp Ser Pro Leu Val Pro
                   165                 170                 175
   caa aaa ggc agt ttt cag atg gtt cac tgc aat tgc agt gtt cat gaa      576
   Gln Lys Gly Ser Phe Gln Met Val His Cys Asn Cys Ser Val His Glu
               180                 185                 190
   tgt tgt gaa tgt ctt gtg cct gtg cca aca gcc aaa ctc aac gac act      624
   Cys Cys Glu Cys Leu Val Pro Val Pro Thr Ala Lys Leu Asn Asp Thr
           195                 200                 205
   ctc ctt atg tgt ttg aaa atc aca tct ggt gga gta att ttc cag tca      672
   Leu Leu Met Cys Leu Lys Ile Thr Ser Gly Gly Val Ile Phe Gln Ser
       210                 215                 220
   cct cta atg tca gtt cag ccc ata aat atg gtg aag cct gat cca cca      720
   Pro Leu Met Ser Val Gln Pro Ile Asn Met Val Lys Pro Asp Pro Pro
   225                 230                 235                 240
   tta ggt ttg cat atg gaa atc aca gat gat ggt aat tta aag att tct      768
   Leu Gly Leu His Met Glu Ile Thr Asp Asp Gly Asn Leu Lys Ile Ser
                   245                 250                 255
   tgg tcc agc cca cca ttg gta cca ttt cca ctt caa tat caa gtg aaa      816
   Trp Ser Ser Pro Pro Leu Val Pro Phe Pro Leu Gln Tyr Gln Val Lys
               260                 265                 270
   tat tca gag aat tct aca aca gtt atc aga gaa gct gac aag att gtc      864
   Tyr Ser Glu Asn Ser Thr Thr Val Ile Arg Glu Ala Asp Lys Ile Val
           275                 280                 285
   tca gct aca tcc ctg cta gta gac agt ata ctt cct ggg tct tcg tat      912
   Ser Ala Thr Ser Leu Leu Val Asp Ser Ile Leu Pro Gly Ser Ser Tyr
       290                 295                 300
   gag gtt cag gtg agg ggc aag aga ctg gat ggc cca gga atc tgg agt      960
   Glu Val Gln Val Arg Gly Lys Arg Leu Asp Gly Pro Gly Ile Trp Ser
   305                 310                 315                 320
   gac tgg agt act cct cgt gtc ttt acc aca caa gat gtc ata tac ttt      1008
   Asp Trp Ser Thr Pro Arg Val Phe Thr Thr Gln Asp Val Ile Tyr Phe
                   325                 330                 335
   cca cct aaa att ctg aca agt gtt ggg tct aat gtt tct ttt cac tgc      1056
   Pro Pro Lys Ile Leu Thr Ser Val Gly Ser Asn Val Ser Phe His Cys
               340                 345                 350
   atc tat aag aag gaa aac aag att gtt ccc tca aaa gag att gtt tgg      1104
   Ile Tyr Lys Lys Glu Asn Lys Ile Val Pro Ser Lys Glu Ile Val Trp
           355                 360                 365
   tgg atg aat tta gct gag aaa att cct caa agc cag tat gat gtt gtg      1152
   Trp Met Asn Leu Ala Glu Lys Ile Pro Gln Ser Gln Tyr Asp Val Val
       370                 375                 380
   agt gat cat gtt agc aaa gtt act ttt ttc aat ctg aat gaa acc aaa    1200
   Ser Asp His Val Ser Lys Val Thr Phe Phe Asn Leu Asn Glu Thr Lys
   385                 390                 395                 400
   cct cga gga aag ttt acc tat gat gca gtg tac tgc tgc aat gaa cat    1248
   Pro Arg Gly Lys Phe Thr Tyr Asp Ala Val Tyr Cys Cys Asn Glu His
                   405                 410                 415
   gaa tgc cat cat cgc tat gct gaa tta tat gtg att gat gtc aat atc    1296
   Glu Cys His His Arg Tyr Ala Glu Leu Tyr Val Ile Asp Val Asn Ile
               420                 425                 430
   aat atc tca tgt gaa act gat ggg tac tta act aaa atg act tgc aga    1344
   Asn Ile Ser Cys Glu Thr Asp Gly Tyr Leu Thr Lys Met Thr Cys Arg
           435                 440                 445
   tgg tca acc agt aca atc cag tca ctt gcg gaa agc act ttg caa ttg    1392
   Trp Ser Thr Ser Thr Ile Gln Ser Leu Ala Glu Ser Thr Leu Gln Leu
       450                 455                 460
   agg tat cat agg agc agc ctt tac tgt tct gat att cca tct att cat    1440
   Arg Tyr His Arg Ser Ser Leu Tyr Cys Ser Asp Ile Pro Ser Ile His
   465                 470                 475                 480
   ccc ata tct gag ccc aaa gat tgc tat ttg cag agt gat ggt ttt tat    1488
   Pro Ile Ser Glu Pro Lys Asp Cys Tyr Leu Gln Ser Asp Gly Phe Tyr
                   485                 490                 495
   gaa tgc att ttc cag cca atc ttc cta tta tct ggc tac aca atg tgg    1536
   Glu Cys Ile Phe Gln Pro Ile Phe Leu Leu Ser Gly Tyr Thr Met Trp
               500                 505                 510
   att agg atc aat cac tct cta ggt tca ctt gac tct cca cca aca tgt    1584
   Ile Arg Ile Asn His Ser Leu Gly Ser Leu Asp Ser Pro Pro Thr Cys
           515                 520                 525
   gtc ctt cct gat tct gtg gtg aag cca ctg cct cca tcc agt gtg aaa    1632
   Val Leu Pro Asp Ser Val Val Lys Pro Leu Pro Pro Ser Ser Val Lys
       530                 535                 540
   gca gaa att act ata aac att gga tta ttg aaa ata tct tgg gaa aag    1680
   Ala Glu Ile Thr Ile Asn Ile Gly Leu Leu Lys Ile Ser Trp Glu Lys
   545                 550                 555                 560
   cca gtc ttt cca gag aat aac ctt caa ttc cag att cgc tat ggt tta    1728
   Pro Val Phe Pro Glu Asn Asn Leu Gln Phe Gln Ile Arg Tyr Gly Leu
                   565                 570                 575
   agt gga aaa gaa gta caa tgg aag atg tat gag gtt tat gat gca aaa    1776
   Ser Gly Lys Glu Val Gln Trp Lys Met Tyr Glu Val Tyr Asp Ala Lys
               580                 585                 590
   tca aaa tct gtc agt ctc cca gtt cca gac ttg tgt gca gtc tat gct    1824
   Ser Lys Ser Val Ser Leu Pro Val Pro Asp Leu Cys Ala Val Tyr Ala
           595                 600                 605
   gtt cag gtg cgc tgt aag agg cta gat gga ctg gga tat tgg agt aat    1872
   Val Gln Val Arg Cys Lys Arg Leu Asp Gly Leu Gly Tyr Trp Ser Asn
       610                 615                 620
   tgg agc aat cca gcc tac aca gtt gtc atg gat ata aaa gtt cct atg    1920
   Trp Ser Asn Pro Ala Tyr Thr Val Val Met Asp Ile Lys Val Pro Met
   625                 630                 635                 640
   aga gga cct gaa ttt tgg aga ata att aat gga gat act atg aaa aag    1968
   Arg Gly Pro Glu Phe Trp Arg Ile Ile Asn Gly Asp Thr Met Lys Lys
                   645                 650                 655
   gag aaa aat gtc act tta ctt tgg aag ccc ctg atg aaa aat gac tca    2016
   Glu Lys Asn Val Thr Leu Leu Trp Lys Pro Leu Met Lys Asn Asp Ser
               660                 665                 670
   ttg tgc agt gtt cag aga tat gtg ata aac cat cat act tcc tgc aat    2064
   Leu Cys Ser Val Gln Arg Tyr Val Ile Asn His His Thr Ser Cys Asn
           675                 680                 685
   gga aca tgg tca gaa gat gtg gga aat cac acg aaa ttc act ttc ctg    2112
   Gly Thr Trp Ser Glu Asp Val Gly Asn His Thr Lys Phe Thr Phe Leu
       690                 695                 700
   tgg aca gag caa gca cat act gtt acg gtt ctg gcc atc aat tca att    2160
   Trp Thr Glu Gln Ala His Thr Val Thr Val Leu Ala Ile Asn Ser Ile
   705                 710                 715                 720
   ggt gct tct gtt gca aat ttt aat tta acc ttt tca tgg cct atg agc    2208
   Gly Ala Ser Val Ala Asn Phe Asn Leu Thr Phe Ser Trp Pro Met Ser
                   725                 730                 735
   aaa gta aat atc gtg cag tca ctc agt gct tat cct tta aac agc agt    2256
   Lys Val Asn Ile Val Gln Ser Leu Ser Ala Tyr Pro Leu Asn Ser Ser
               740                 745                 750
   tgt gtg att gtt tcc tgg ata cta tca ccc agt gat tac aag cta atg    2304
   Cys Val Ile Val Ser Trp Ile Leu Ser Pro Ser Asp Tyr Lys Leu Met
           755                 760                 765
   tat ttt att att gag tgg aaa aat ctt aat gaa gat ggt gaa ata aaa    2352
   Tyr Phe Ile Ile Glu Trp Lys Asn Leu Asn Glu Asp Gly Glu Ile Lys
       770                 775                 780
   tgg ctt aga atc tct tca tct gtt aag aag tat tat atc cat gat cat    2400
   Trp Leu Arg Ile Ser Ser Ser Val Lys Lys Tyr Tyr Ile His Asp His
   785                 790                 795                 800
   ttt atc ccc att gag aag tac cag ttc agt ctt tac cca ata ttt atg    2448
   Phe Ile Pro Ile Glu Lys Tyr Gln Phe Ser Leu Tyr Pro Ile Phe Met
                   805                 810                 815
   gaa gga gtg gga aaa cca aag ata att aat agt ttc act caa gat gat    2496
   Glu Gly Val Gly Lys Pro Lys Ile Ile Asn Ser Phe Thr Gln Asp Asp
               820                 825                 830
   att gaa aaa cac cag agt gat gca ggt tta tat gta att gtg cca gta    2544
   Ile Glu Lys His Gln Ser Asp Ala Gly Leu Tyr Val Ile Val Pro Val
           835                 840                 845
   att att tcc tct tcc atc tta ttg ctt gga aca tta tta ata tca cac    2592
   Ile Ile Ser Ser Ser Ile Leu Leu Leu Gly Thr Leu Leu Ile Ser His
       850                 855                 860
   caa aga atg aaa aag cta ttt tgg gaa gat gtt ccg aac ccc aag aat    2640
   Gln Arg Met Lys Lys Leu Phe Trp Glu Asp Val Pro Asn Pro Lys Asn
   865                 870                     875                 880
   tgt tcc tgg gca caa gga ctt aat ttt cag aag aga acg gac att ctt      2688
   Cys Ser Trp Ala Gln Gly Leu Asn Phe Gln Lys Arg Thr Asp Ile Leu
                   885                 890                 895
   tga                                                                  2691
<210> 2
   <211> 896
   <212> PRT
   <213> Homo sapiens
<400> 2
   Met Ile Cys Gln Lys Phe Cys Val Val Leu Leu His Trp Glu Phe Ile
   1 5 10 15
   Tyr Val Ile Thr Ala Phe Asn Leu Ser Tyr Pro Ile Thr Pro Trp Arg
   20 25 30
   Phe Lys Leu Ser Cys Met Pro Pro Asn Ser Thr Tyr Asp Tyr Phe Leu
   35 40 45
   Leu Pro Ala Gly Leu Ser Lys Asn Thr Ser Asn Ser Asn Gly His Tyr
   50 55 60
   Glu Thr Ala Val Glu Pro Lys Phe Asn Ser Ser Gly Thr His Phe Ser
   65 70 75 80
   Asn Leu Ser Lys Thr Thr Phe His Cys Cys Phe Arg Ser Glu Gln Asp
   85 90 95
   Arg Asn Cys Ser Leu Cys Ala Asp Asn Ile Glu Gly Lys Thr Phe Val
   100 105 110
   Ser Thr Val Asn Ser Leu Val Phe Gln Gln Ile Asp Ala Asn Trp Asn
   115 120 125
   Ile Gln Cys Trp Leu Lys Gly Asp Leu Lys Leu Phe Ile Cys Tyr Val
   130 135 140
   Glu Ser Leu Phe Lys Asn Leu Phe Arg Asn Tyr Asn Tyr Lys Val His
   145 150 155 160
   Leu Leu Tyr Val Leu Pro Glu Val Leu Glu Asp Ser Pro Leu Val Pro
   165 170 175
   Gln Lys Gly Ser Phe Gln Met Val His Cys Asn Cys Ser Val His Glu
   180 185 190
   Cys Cys Glu Cys Leu Val Pro Val Pro Thr Ala Lys Leu Asn Asp Thr
   195 200 205
   Leu Leu Met Cys Leu Lys Ile Thr Ser Gly Gly Val Ile Phe Gln Ser
   210 215 220
   Pro Leu Met Ser Val Gln Pro Ile Asn Met Val Lys Pro Asp Pro Pro
   225 230 235 240
   Leu Gly Leu His Met Glu Ile Thr Asp Asp Gly Asn Leu Lys Ile Ser
   245 250 255
   Trp Ser Ser Pro Pro Leu Val Pro Phe Pro Leu Gln Tyr Gln Val Lys
   260 265 270
   Tyr Ser Glu Asn Ser Thr Thr Val Ile Arg Glu Ala Asp Lys Ile Val
   275 280 285
   Ser Ala Thr Ser Leu Leu Val Asp Ser Ile Leu Pro Gly Ser Ser Tyr
   290 295 300
   Glu Val Gln Val Arg Gly Lys Arg Leu Asp Gly Pro Gly Ile Trp Ser
   305 310 315 320
   Asp Trp Ser Thr Pro Arg Val Phe Thr Thr Gln Asp Val Ile Tyr Phe
   325 330 335
   Pro Pro Lys Ile Leu Thr Ser Val Gly Ser Asn Val Ser Phe His Cys
   340 345 350
   Ile Tyr Lys Lys Glu Asn Lys Ile Val Pro Ser Lys Glu Ile Val Trp
   355 360 365
   Trp Met Asn Leu Ala Glu Lys Ile Pro Gln Ser Gln Tyr Asp Val Val
   370 375 380
   Ser Asp His Val Ser Lys Val Thr Phe Phe Asn Leu Asn Glu Thr Lys
   385 390 395 400
   Pro Arg Gly Lys Phe Thr Tyr Asp Ala Val Tyr Cys Cys Asn Glu His
   405 410 415
   Glu Cys His His Arg Tyr Ala Glu Leu Tyr Val Ile Asp Val Asn Ile
   420 425 430
   Asn Ile Ser Cys Glu Thr Asp Gly Tyr Leu Thr Lys Met Thr Cys Arg
   435 440 445
   Trp Ser Thr Ser Thr Ile Gln Ser Leu Ala Glu Ser Thr Leu Gln Leu
   450 455 460
   Arg Tyr His Arg Ser Ser Leu Tyr Cys Ser Asp Ile Pro Ser Ile His
   465 470 475 480
   Pro Ile Ser Glu Pro Lys Asp Cys Tyr Leu Gln Ser Asp Gly Phe Tyr
   485 490 495
   Glu Cys Ile Phe Gln Pro Ile Phe Leu Leu Ser Gly Tyr Thr Met Trp
   500 505 510
   Ile Arg Ile Asn His Ser Leu Gly Ser Leu Asp Ser Pro Pro Thr Cys
   515 520 525
   Val Leu Pro Asp Ser Val Val Lys Pro Leu Pro Pro Ser Ser Val Lys
   530 535 540
   Ala Glu Ile Thr Ile Asn Ile Gly Leu Leu Lys Ile Ser Trp Glu Lys
   545 550 555 560
   Pro Val Phe Pro Glu Asn Asn Leu Gln Phe Gln Ile Arg Tyr Gly Leu
   565 570 575
   Ser Gly Lys Glu Val Gln Trp Lys Met Tyr Glu Val Tyr Asp Ala Lys
   580 585 590
   Ser Lys Ser Val Ser Leu Pro Val Pro Asp Leu Cys Ala Val Tyr Ala
   595 600 605
   Val Gln Val Arg Cys Lys Arg Leu Asp Gly Leu Gly Tyr Trp Ser Asn
   610 615 620
   Trp Ser Asn Pro Ala Tyr Thr Val Val Met Asp Ile Lys Val Pro Met
   625 630 635 640
   Arg Gly Pro Glu Phe Trp Arg Ile Ile Asn Gly Asp Thr Met Lys Lys
   645 650 655
   Glu Lys Asn Val Thr Leu Leu Trp Lys Pro Leu Met Lys Asn Asp Ser
   660 665 670
   Leu Cys Ser Val Gln Arg Tyr Val Ile Asn His His Thr Ser Cys Asn
   675 680 685
   Gly Thr Trp Ser Glu Asp Val Gly Asn His Thr Lys Phe Thr Phe Leu
   690 695 700
   Trp Thr Glu Gln Ala His Thr Val Thr Val Leu Ala Ile Asn Ser Ile
   705 710 715 720
   Gly Ala Ser Val Ala Asn Phe Asn Leu Thr Phe Ser Trp Pro Met Ser
   725 730 735
   Lys Val Asn Ile Val Gln Ser Leu Ser Ala Tyr Pro Leu Asn Ser Ser
   740 745 750
   Cys Val Ile Val Ser Trp Ile Leu Ser Pro Ser Asp Tyr Lys Leu Met
   755 760 765
   Tyr Phe Ile Ile Glu Trp Lys Asn Leu Asn Glu Asp Gly Glu Ile Lys
   770 775 780
   Trp Leu Arg Ile Ser Ser Ser Val Lys Lys Tyr Tyr Ile His Asp His
   785 790 795 800
   Phe Ile Pro Ile Glu Lys Tyr Gln Phe Ser Leu Tyr Pro Ile Phe Met
   805 810 815
   Glu Gly Val Gly Lys Pro Lys Ile Ile Asn Ser Phe Thr Gln Asp Asp
   820 825 830
   Ile Glu Lys His Gln Ser Asp Ala Gly Leu Tyr Val Ile Val Pro Val
   835 840 845
   Ile Ile Ser Ser Ser Ile Leu Leu Leu Gly Thr Leu Leu Ile Ser His
   850 855 860
   Gln Arg Met Lys Lys Leu Phe Trp Glu Asp Val Pro Asn Pro Lys Asn
   865 870 875 880
   Cys Ser Trp Ala Gln Gly Leu Asn Phe Gln Lys Arg Thr Asp Ile Leu
<210> 3
   <211> 2751
   <212> ADN
   <213> Séquence artificielle
<220>
   <221> CDS
   <222> (1)..(2757)
<220>
   <223> Description de la séquence artificielle:Protéine de fusion comprenant une partie d'OBRs
<400> 3
   atg gtt ctt gcc agc tct acc acc agc atc cac acc atg ctg ctc ctg      48
   Met Val Leu Ala Ser Ser Thr Thr Ser Ile His Thr Met Leu Leu Leu
    1               5                  10                  15
   ctc ctg atg ctc ttc cac ctg gga ctc caa gct tca atc tcg gcg cgc      96
   Leu Leu Met Leu Phe His Leu Gly Leu Gln Ala Ser Ile Ser Ala Arg
               20                  25                  30
   cag gag cag aag ctt atc tcg gag gag gac ctg acg cgt tat cca att     144
   Gln Glu Gln Lys Leu Ile Ser Glu Glu Asp Leu Thr Arg Tyr Pro Ile
           35                  40                  45
   act cct tgg aga ttt aag ttg tct tgc atg cca cca aat tca acc tat     192
   Thr Pro Trp Arg Phe Lys Leu Ser Cys Met Pro Pro Asn Ser Thr Tyr
       50                  55                  60
   gac tac ttc ctt ttg cct gct gga ctc tca aag aat act tca aat tcg     240
   Asp Tyr Phe Leu Leu Pro Ala Gly Leu Ser Lys Asn Thr Ser Asn Ser
   65                  70                  75                  80
   aat gga cat tat gag aca gct gtt gaa cct aag ttt aat tca agt ggt     288
   Asn Gly His Tyr Glu Thr Ala Val Glu Pro Lys Phe Asn Ser Ser Gly
                   85                  90                  95
   act cac ttt tct aac tta tcc aaa aca act ttc cac tgt tgc ttt cgg     336
   Thr His Phe Ser Asn Leu Ser Lys Thr Thr Phe His Cys Cys Phe Arg
               100                 105                 110
   agt gag caa gat aga aac tgc tcc tta tgt gca gac aac att gaa gga     384
   Ser Glu Gln Asp Arg Asn Cys Ser Leu Cys Ala Asp Asn Ile Glu Gly
           115                 120                 125
   acg aca ttt gtt tca aca gta aat tct tta gtt ttt caa caa ata gat     432
   Thr Thr Phe Val Ser Thr Val Asn Ser Leu Val Phe Gln Gln Ile Asp
       130                 135                 140
   gca aac tgg aac ata cag tgc tgg cta aaa gga gac tta aaa tta ttc     480
   Ala Asn Trp Asn Ile Gln Cys Trp Leu Lys Gly Asp Leu Lys Leu Phe
   145                 150                 155                 160
   atc tgt tat gtg gag tca tta ttt aag aat cta ttc agg aat tat aac     528
   Ile Cys Tyr Val Glu Ser Leu Phe Lys Asn Leu Phe Arg Asn Tyr Asn
                   165                 170                 175
   tat aag gtc cat ctt tta tat gtt ctg cct gaa gtg tta gaa gat tca     576
   Tyr Lys Val His Leu Leu Tyr Val Leu Pro Glu Val Leu Glu Asp Ser
               180                 185                 190
   cct ctg gtt ccc caa aaa ggc agt ttt cag atg gtt cac tgc aat tgc     624
   Pro Leu Val Pro Gln Lys Gly Ser Phe Gln Met Val His Cys Asn Cys
           195                 200                 205
   agt gtt cat gaa tgt tgt gaa tgt ctt gtg cct gtg cca aca gcc aaa     672
   Ser Val His Glu Cys Cys Glu Cys Leu Val Pro Val Pro Thr Ala Lys
       210                 215                 220
   ctc aac gac act ctc ctt atg tgt ttg aaa atc aca tct ggt gga gta     720
   Leu Asn Asp Thr Leu Leu Met Cys Leu Lys Ile Thr Ser Gly Gly Val
   225                 230                 235                 240
   att ttc cgg tca cct cta atg tca gtt cag ccc ata aat atg gtg aag     768
   Ile Phe Arg Ser Pro Leu Met Ser Val Gln Pro Ile Asn Met Val Lys
                   245                 250                 255
   cct gat cca cca tta ggt ttg cat atg gaa atc aca gat gat ggt aat     816
   Pro Asp Pro Pro Leu Gly Leu His Met Glu Ile Thr Asp Asp Gly Asn
               260                 265                 270
   tta aag att tct tgg tcc agc cca cca ttg gta cca ttt cca ctt caa     864
   Leu Lys Ile Ser Trp Ser Ser Pro Pro Leu Val Pro Phe Pro Leu Gln
           275                 280                 285
   tat caa gtg aaa tat tca gag aat tct aca aca gtt atc aga gaa gct     912
   Tyr Gln Val Lys Tyr Ser Glu Asn Ser Thr Thr Val Ile Arg Glu Ala
       290                 295                 300
   gac aag att gtc tca gct aca tcc ctg cta gta gac agt ata ctt cct     960
   Asp Lys Ile Val Ser Ala Thr Ser Leu Leu Val Asp Ser Ile Leu Pro
   305                 310                 315                 320
   ggg tct tcg tat gag gtt cag gtg agg ggc aag aga ctg gat ggc cca     1008
   Gly Ser Ser Tyr Glu Val Gln Val Arg Gly Lys Arg Leu Asp Gly Pro
                   325                 330                 335
   gga atc tgg agt gac tgg agt act cct cgt gtc ttt acc aca caa gat     1056
   Gly Ile Trp Ser Asp Trp Ser Thr Pro Arg Val Phe Thr Thr Gln Asp
               340                 345                 350
   gtc ata tac ttt cca cct aaa att ctg aca agt gtt ggg tct aat gtt     1104
   Val Ile Tyr Phe Pro Pro Lys Ile Leu Thr Ser Val Gly Ser Asn Val
           355                 360                 365
   tct ttt cac tgc atc tat aag aag gaa aac aag att gtt ccc tca aaa     1152
   Ser Phe His Cys Ile Tyr Lys Lys Glu Asn Lys Ile Val Pro Ser Lys
       370                 375                 380
   gag att gtt tgg tgg atg aat tta gct gag aaa att cct caa agc cag     1200
   Glu Ile Val Trp Trp Met Asn Leu Ala Glu Lys Ile Pro Gln Ser Gln
   385                 390                 395                 400
   tat gat gtt gtg agt gat cat gtt agc aaa gtt act ttt ttc aat ctg     1248
   Tyr Asp Val Val Ser Asp His Val Ser Lys Val Thr Phe Phe Asn Leu
                   405                 410                 415
   aat gaa acc aaa cct cga gga aag ttt acc tat gat gca gtg tac tgc    1296
   Asn Glu Thr Lys Pro Arg Gly Lys Phe Thr Tyr Asp Ala Val Tyr Cys
               420                 425                 430
   tgc aat gaa cat gaa tgc cat cat cgc tat gct gaa tta tat gtg att    1344
   Cys Asn Glu His Glu Cys His His Arg Tyr Ala Glu Leu Tyr Val Ile
           435                 440                 445
   gat gtc aat atc aat atc tca tgt gaa act gat ggg tac tta act aaa    1392
   Asp Val Asn Ile Asn Ile Ser Cys Glu Thr Asp Gly Tyr Leu Thr Lys
       450                 455                 460
   atg act tgc aga tgg tca acc agt aca atc cag tca ctt gcg gaa agc    1440
   Met Thr Cys Arg Trp Ser Thr Ser Thr Ile Gln Ser Leu Ala Glu Ser
   465                 470                 475                 480
   act ttg caa ttg agg tat cat agg agc agc ctt tac tgt tct gat att    1488
   Thr Leu Gln Leu Arg Tyr His Arg Ser Ser Leu Tyr Cys Ser Asp Ile
                   485                 490                 495
   cca tct att cat ccc ata tct gag ccc aaa gat tgc tat ttg cag agt    1536
   Pro Ser Ile His Pro Ile Ser Glu Pro Lys Asp Cys Tyr Leu Gln Ser
               500                 505                 510
   gat ggt ttt tat gaa tgc att ttc cag cca atc ttc cta tta tct ggc    1584
   Asp Gly Phe Tyr Glu Cys Ile Phe Gln Pro Ile Phe Leu Leu Ser Gly
           515                 520                 525
   tac aca atg tgg att agg atc aat cac tct cta ggt tca ctt gac tct    1632
   Tyr Thr Met Trp Ile Arg Ile Asn His Ser Leu Gly Ser Leu Asp Ser
       530                 535                 540
   cca cca aca tgt gtc ctt cct gat tct gtg gtg aag cca ctg cct cca    1680
   Pro Pro Thr Cys Val Leu Pro Asp Ser Val Val Lys Pro Leu Pro Pro
   545                 550                 555                 560
   tcc agt gtg aaa gca gaa att act ata aac att gga tta ttg aaa ata    1728
   Ser Ser Val Lys Ala Glu Ile Thr Ile Asn Ile Gly Leu Leu Lys Ile
                   565                 570                 575
   tct tgg gaa aag cca gtc ttt cca gag aat aac ctt caa ttc cag att    1776
   Ser Trp Glu Lys Pro Val Phe Pro Glu Asn Asn Leu Gln Phe Gln Ile
               580                 585                 590
   cgc tat ggt tta agt gga aaa gaa gta caa tgg aag atg tat gag gtt    1824
   Arg Tyr Gly Leu Ser Gly Lys Glu Val Gln Trp Lys Met Tyr Glu Val
           595                 600                 605
   tat gat gca aaa tca aaa tct gtc agt ctc cca gtt cca gac ttg tgt    1872
   Tyr Asp Ala Lys Ser Lys Ser Val Ser Leu Pro Val Pro Asp Leu Cys
       610                 615                 620
   gca gtc tat gct gtt cag gtg cgc tgt aag agg cta gat gga ctg gga    1920
   Ala Val Tyr Ala Val Gln Val Arg Cys Lys Arg Leu Asp Gly Leu Gly
   625                 630                 635                 640
   tat tgg agt aat tgg agc aat cca gcc tac aca gtt gtc atg gat ata    1968
   Tyr Trp Ser Asn Trp Ser Asn Pro Ala Tyr Thr Val Val Met Asp Ile
                   645                 650                 655
   aaa gtt cct atg aga gga cct gaa ttt tgg aga ata att aat gga gat    2016
   Lys Val Pro Met Arg Gly Pro Glu Phe Trp Arg Ile Ile Asn Gly Asp
   660 665 670
   act atg aaa aag gag aaa aat gtc act tta ctt tgg aag ccc ctg atg 2064
   Thr Met Lys Lys Glu Lys Asn Val Thr Leu Leu Trp Lys Pro Leu Met
   675 680 685
   aaa aat gac tca ttg tgc agt gtt cag aga tat gtg ata aac cat cat 2112
   Lys Asn Asp Ser Leu Cys Ser Val Gln Arg Tyr Val Ile Asn His His
   690 695 700
   act tcc tgc aat gga aca tgg tca gaa gat gtg gga aat cac acg aaa 2160
   Thr Ser Cys Asn Gly Thr Trp Ser Glu Asp Val Gly Asn His Thr Lys
   705 710 715 720
   ttc act ttc ctg tgg aca gag caa gca cat act gtt acg gtt ctg gcc 2208
   Phe Thr Phe Leu Trp Thr Glu Gln Ala His Thr Val Thr Val Leu Ala
   725 730 735
   atc aat tca att ggt gct tct gtt gca aat ttt aat tta acc ttt tca 2256
   Ile Asn Ser Ile Gly Ala Ser Val Ala Asn Phe Asn Leu Thr Phe Ser
   740 745 750
   tgg cct atg agc aaa gta aat atc gtg cag tca ctc agt gct tat cct 2304
   Trp Pro Met Ser Lys Val Asn Ile Val Gln Ser Leu Ser Ala Tyr Pro
   755 760 765
   tta aac agc agt tgt gtg att gtt tcc tgg ata cta tca ccc agt gat 2352
   Leu Asn Ser Ser Cys Val Ile Val Ser Trp Ile Leu Ser Pro Ser Asp
   770 775 780
   tac aag cta atg tat ttt att att gag tgg aaa aat ctt aat gaa gat 2400
   Tyr Lys Leu Met Tyr Phe Ile Ile Glu Trp Lys Asn Leu Asn Glu Asp
   785 790 795 800
   ggt gaa ata aaa tgg ctt aga atc tct tca tct gtt aag aag tat tat 2448
   Gly Glu Ile Lys Trp Leu Arg Ile Ser Ser Ser Val Lys Lys Tyr Tyr
   805 810 815
   atc cat gat cat ttt atc ccc att gag aag tac cag ttc agt ctt tac 2496
   Ile His Asp His Phe Ile Pro Ile Glu Lys Tyr Gln Phe Ser Leu Tyr
   820 825 830
   cca ata ttt atg gaa gga gtg gga aaa cca aag ata att aat agt ttc 2544
   Pro Ile Phe Met Glu Gly Val Gly Lys Pro Lys Ile Ile Asn Ser Phe
   835 840 845
   act caa gat gat att gaa aaa cac cag agt gat gca ggt tta tat gta 2592
   Thr Gln Asp Asp Ile Glu Lys His Gln Ser Asp Ala Gly Leu Tyr Val
   850 855 860
   att gtg cca gta att att tcc tct tcc atc tta ttg ctt gga aca tta 2640
   Ile Val Pro Val Ile Ile Ser Ser Ser Ile Leu Leu Leu Gly Thr Leu
   865 870 875 880
   tta ata tca cac caa aga atg aaa aag cta ttt tgg gaa gat gtt ccg 2688
   Leu Ile Ser His Gln Arg Met Lys Lys Leu Phe Trp Glu Asp Val Pro
   885 890 895
   aac ccc aag aat tgt tcc tgg gca caa gga ctt aat ttt cag aag aga 2736
   Asn Pro Lys Asn Cys Ser Trp Ala Gln Gly Leu Asn Phe Gln Lys Arg
   900 905 910
   acg gac att ctt tga 2751
   Thr Asp Ile Leu
   915
<210> 4
   <211> 916
   <212> PRT
   <213> Séquence artificielle
   <223> Description de la séquence artificielle:Protéine de fusion comprenant une partie d'OBRs
<400> 4
   Met Val Leu Ala Ser Ser Thr Thr Ser Ile His Thr Met Leu Leu Leu
   1               5                   10                  15
   Leu Leu Met Leu Phe His Leu Gly Leu Gln Ala Ser Ile Ser Ala Arg
               20                  25                  30
   Gln Glu Gln Lys Leu Ile Ser Glu Glu Asp Leu Thr Arg Tyr Pro Ile
           35                  40                  45
   Thr Pro Trp Arg Phe Lys Leu Ser Cys Met Pro Pro Asn Ser Thr Tyr
       50                  55                  60
   Asp Tyr Phe Leu Leu Pro Ala Gly Leu Ser Lys Asn Thr Ser Asn Ser
   65                  70                  75                  80
   Asn Gly His Tyr Glu Thr Ala Val Glu Pro Lys Phe Asn Ser Ser Gly
                   85                  90                  95
   Thr His Phe Ser Asn Leu Ser Lys Thr Thr Phe His Cys Cys Phe Arg
               100                 105                 110
   Ser Glu Gln Asp Arg Asn Cys Ser Leu Cys Ala Asp Asn Ile Glu Gly
           115                 120                 125
   Thr Thr Phe Val Ser Thr Val Asn Ser Leu Val Phe Gln Gln Ile Asp
       130                 135                 140
   Ala Asn Trp Asn Ile Gln Cys Trp Leu Lys Gly Asp Leu Lys Leu Phe
   145                 150                 155                 160
   Ile Cys Tyr Val Glu Ser Leu Phe Lys Asn Leu Phe Arg Asn Tyr Asn
                   165                 170                 175
   Tyr Lys Val His Leu Leu Tyr Val Leu Pro Glu Val Leu Glu Asp Ser
               180                 185                 190
   Pro Leu Val Pro Gln Lys Gly Ser Phe Gln Met Val His Cys Asn Cys
           195                 200                 205
   Ser Val His Glu Cys Cys Glu Cys Leu Val Pro Val Pro Thr Ala Lys
       210                 215                 220
   Leu Asn Asp Thr Leu Leu Met Cys Leu Lys Ile Thr Ser Gly Gly Val
   225                 230                 235                 240
   Ile Phe Arg Ser Pro Leu Met Ser Val Gln Pro Ile Asn Met Val Lys
                       245                 250                 255
   Pro Asp Pro Pro Leu Gly Leu His Met Glu Ile Thr Asp Asp Gly Asn
               260                 265                 270
   Leu Lys Ile Ser Trp Ser Ser Pro Pro Leu Val Pro Phe Pro Leu Gln
           275                 280                 285
   Tyr Gln Val Lys Tyr Ser Glu Asn Ser Thr Thr Val Ile Arg Glu Ala
       290                 295                 300
   Asp Lys Ile Val Ser Ala Thr Ser Leu Leu Val Asp Ser Ile Leu Pro
   305                 310                 315                 320
   Gly Ser Ser Tyr Glu Val Gln Val Arg Gly Lys Arg Leu Asp Gly Pro
                   325                 330                 335
   Gly Ile Trp Ser Asp Trp Ser Thr Pro Arg Val Phe Thr Thr Gln Asp
               340                 345                 350
   Val Ile Tyr Phe Pro Pro Lys Ile Leu Thr Ser Val Gly Ser Asn Val
           355                 360                 365
   Ser Phe His Cys Ile Tyr Lys Lys Glu Asn Lys Ile Val Pro Ser Lys
       370                 375                 380
   Glu Ile Val Trp Trp Met Asn Leu Ala Glu Lys Ile Pro Gln Ser Gln
   385                 390                 395                 400
   Tyr Asp Val Val Ser Asp His Val Ser Lys Val Thr Phe Phe Asn Leu
                   405                 410                 415
   Asn Glu Thr Lys Pro Arg Gly Lys Phe Thr Tyr Asp Ala Val Tyr Cys
               420                 425                 430
   Cys Asn Glu His Glu Cys His His Arg Tyr Ala Glu Leu Tyr Val Ile
           435                 440                 445
   Asp Val Asn Ile Asn Ile Ser Cys Glu Thr Asp Gly Tyr Leu Thr Lys
       450                 455                 460
   Met Thr Cys Arg Trp Ser Thr Ser Thr Ile Gln Ser Leu Ala Glu Ser
   465                 470                 475                 480
   Thr Leu Gln Leu Arg Tyr His Arg Ser Ser Leu Tyr Cys Ser Asp Ile
                   485                 490                 495
   Pro Ser Ile His Pro Ile Ser Glu Pro Lys Asp Cys Tyr Leu Gln Ser
               500                 505                 510
   Asp Gly Phe Tyr Glu Cys Ile Phe Gln Pro Ile Phe Leu Leu Ser Gly
           515                 520                 525
   Tyr Thr Met Trp Ile Arg Ile Asn His Ser Leu Gly Ser Leu Asp Ser
       530                 535                 540
   Pro Pro Thr Cys Val Leu Pro Asp Ser Val Val Lys Pro Leu Pro Pro
   545                 550                 555                 560
   Ser Ser Val Lys Ala Glu Ile Thr Ile Asn Ile Gly Leu Leu Lys Ile
                   565                 570                 575
   Ser Trp Glu Lys Pro Val Phe Pro Glu Asn Asn Leu Gln Phe Gln Ile
               580                 585                 590
   Arg Tyr Gly Leu Ser Gly Lys Glu Val Gln Trp Lys Met Tyr Glu Val
           595                 600                 605
   Tyr Asp Ala Lys Ser Lys Ser Val Ser Leu Pro Val Pro Asp Leu Cys
       610                 615                 620
   Ala Val Tyr Ala Val Gln Val Arg Cys Lys Arg Leu Asp Gly Leu Gly
   625                 630                 635                 640
   Tyr Trp Ser Asn Trp Ser Asn Pro Ala Tyr Thr Val Val Met Asp Ile
                   645                 650                 655
   Lys Val Pro Met Arg Gly Pro Glu Phe Trp Arg Ile Ile Asn Gly Asp
               660                 665                 670
   Thr Met Lys Lys Glu Lys Asn Val Thr Leu Leu Trp Lys Pro Leu Met
           675                 680                 685
   Lys Asn Asp Ser Leu Cys Ser Val Gln Arg Tyr Val Ile Asn His His
       690                 695                 700
   Thr Ser Cys Asn Gly Thr Trp Ser Glu Asp Val Gly Asn His Thr Lys
   705                 710                 715                 720
   Phe Thr Phe Leu Trp Thr Glu Gln Ala His Thr Val Thr Val Leu Ala
                   725                 730                 735
   Ile Asn Ser Ile Gly Ala Ser Val Ala Asn Phe Asn Leu Thr Phe Ser
               740                 745                 750
   Trp Pro Met Ser Lys Val Asn Ile Val Gln Ser Leu Ser Ala Tyr Pro
           755                 760                 765
   Leu Asn Ser Ser Cys Val Ile Val Ser Trp Ile Leu Ser Pro Ser Asp
       770                 775                 780
   Tyr Lys Leu Met Tyr Phe Ile Ile Glu Trp Lys Asn Leu Asn Glu Asp
   785                 790                 795                 800
   Gly Glu Ile Lys Trp Leu Arg Ile Ser Ser Ser Val Lys Lys Tyr Tyr
                   805                 810                 815
   Ile His Asp His Phe Ile Pro Ile Glu Lys Tyr Gln Phe Ser Leu Tyr
               820                 825                 830
   Pro Ile Phe Met Glu Gly Val Gly Lys Pro Lys Ile Ile Asn Ser Phe
           835                 840                 845
   Thr Gln Asp Asp Ile Glu Lys His Gln Ser Asp Ala Gly Leu Tyr Val
       850                 855                 860
   Ile Val Pro Val Ile Ile Ser Ser Ser Ile Leu Leu Leu Gly Thr Leu
   865                 870                 875                 880
   Leu Ile Ser His Gln Arg Met Lys Lys Leu Phe Trp Glu Asp Val Pro
                   885                 890                 895
   Asn Pro Lys Asn Cys Ser Trp Ala Gln Gly Leu Asn Phe Gln Lys Arg
   900 905 910
   Thr Asp Ile Leu
   915
<210> 5
   <211> 3705
   <212> ADN
   <213> Séquence artificielle
<220>
   <223> Description de la séquence artificielle:fusionOBRluc
<220>
   <221> CDS
   <222> (1)..(3705)
<400> 5
   atg gtt ctt gcc agc tct acc acc agc atc cac acc atg ctg ctc ctg    48
   ctc ctg atg ctc ttc cac ctg gga ctc caa gct tca atc tcg gcg cgc    96
   cag gag cag aag ctt atc tcg gag gag gac ctg acg cgt tat cca att    144
   act cct tgg aga ttt aag ttg tct tgc atg cca cca aat tca acc tat    192
   gac tac ttc ctt ttg cct gct gga ctc tca aag aat act tca aat tcg    240
   aat gga cat tat gag aca gct gtt gaa cct aag ttt aat tca agt ggt    288
   act cac ttt tct aac tta tcc aaa aca act ttc cac tgt tgc ttt cgg    336
   agt gag caa gat aga aac tgc tcc tta tgt gca gac aac att gaa gga    384
   acg aca ttt gtt tca aca gta aat tct tta gtt ttt caa caa ata gat    432
   gca aac tgg aac ata cag tgc tgg cta aaa gga gac tta aaa tta ttc    480
   atc tgt tat gtg gag tca tta ttt aag aat cta ttc agg aat tat aac     528
   tat aag gtc cat ctt tta tat gtt ctg cct gaa gtg tta gaa gat tca     576
   cct ctg gtt ccc caa aaa ggc agt ttt cag atg gtt cac tgc aat tgc     624
   agt gtt cat gaa tgt tgt gaa tgt ctt gtg cct gtg cca aca gcc aaa     672
   ctc aac gac act ctc ctt atg tgt ttg aaa atc aca tct ggt gga gta     720
   att ttc cgg tca cct cta atg tca gtt cag ccc ata aat atg gtg aag     768
   cct gat cca cca tta ggt ttg cat atg gaa atc aca gat gat ggt aat     816
   tta aag att tct tgg tcc agc cca cca ttg gta cca ttt cca ctt caa     864
   tat caa gtg aaa tat tca gag aat tct aca aca gtt atc aga gaa gct     912
   gac aag att gtc tca gct aca tcc ctg cta gta gac agt ata ctt cct     960
   ggg tct tcg tat gag gtt cag gtg agg ggc aag aga ctg gat ggc cca    1008
   gga atc tgg agt gac tgg agt act cct cgt gtc ttt acc aca caa gat    1056
   gtc ata tac ttt cca cct aaa att ctg aca agt gtt ggg tct aat gtt    1104
   tct ttt cac tgc atc tat aag aag gaa aac aag att gtt ccc tca aaa    1152
   gag att gtt tgg tgg atg aat tta gct gag aaa att cct caa agc cag    1200
   tat gat gtt gtg agt gat cat gtt agc aaa gtt act ttt ttc aat ctg 1248
   aat gaa acc aaa cct cga gga aag ttt acc tat gat gca gtg tac tgc 1296
   tgc aat gaa cat gaa tgc cat cat cgc tat gct gaa tta tat gtg att 1344
   gat gtc aat atc aat atc tca tgt gaa act gat ggg tac tta act aaa 1392
   atg act tgc aga tgg tca acc agt aca atc cag tca ctt gcg gaa agc 1440
   act ttg caa ttg agg tat cat agg agc agc ctt tac tgt tct gat att 1488
   cca tct att cat ccc ata tct gag ccc aaa gat tgc tat ttg cag agt 1536
   gat ggt ttt tat gaa tgc att ttc cag cca atc ttc cta tta tct ggc 1584
   tac aca atg tgg att agg atc aat cac tct cta ggt tca ctt gac tct 1632
   cca cca aca tgt gtc ctt cct gat tct gtg gtg aag cca ctg cct cca 1680
   tcc agt gtg aaa gca gaa att act ata aac att gga tta ttg aaa ata 1728
   tct tgg gaa aag cca gtc ttt cca gag aat aac ctt caa ttc cag att 1776
   cgc tat ggt tta agt gga aaa gaa gta caa tgg aag atg tat gag gtt 1824
   tat gat gca aaa tca aaa tct gtc agt ctc cca gtt cca gac ttg tgt 1872
   gca gtc tat gct gtt cag gtg cgc tgt aag agg cta gat gga ctg gga 1920
   tat tgg agt aat tgg agc aat cca gcc tac aca gtt gtc atg gat ata 1968
   aaa gtt cct atg aga gga cct gaa ttt tgg aga ata att aat gga gat      2016
   act atg aaa aag gag aaa aat gtc act tta ctt tgg aag ccc ctg atg      2064
   aaa aat gac tca ttg tgc agt gtt cag aga tat gtg ata aac cat cat      2112
   act tcc tgc aat gga aca tgg tca gaa gat gtg gga aat cac acg aaa      2160
   ttc act ttc ctg tgg aca gag caa gca cat act gtt acg gtt ctg gcc      2208
   atc aat tca att ggt gct tct gtt gca aat ttt aat tta acc ttt tca      2256
   tgg cct atg agc aaa gta aat atc gtg cag tca ctc agt gct tat cct      2304
   tta aac agc agt tgt gtg att gtt tcc tgg ata cta tca ccc agt gat      2352
   tac aag cta atg tat ttt att att gag tgg aaa aat ctt aat gaa gat      2400
   ggt gaa ata aaa tgg ctt aga atc tct tca tct gtt aag aag tat tat      2448
   atc cat gat cat ttt atc ccc att gag aag tac cag ttc agt ctt tac      2496
   cca ata ttt atg gaa gga gtg gga aaa cca aag ata att aat agt ttc      2544
   act caa gat gat att gaa aaa cac cag agt gat gca ggt tta tat gta      2592
   att gtg cca gta att att tcc tct tcc atc tta ttg ctt gga aca tta      2640
   tta ata tca cac caa aga atg aaa aag cta ttt tgg gaa gat gtt ccg      2688
   aac ccc aag aat tgt tcc tgg gca caa gga ctt aat ttt cag aag aga    2736
   acg gac att ctg gat cca ccg gct aga gcc acc atg acc agc aag gtg    2784
   tac gac ccc gag cag agg aag agg atg atc acc ggc ccc cag tgg tgg    2832
   gcc agg tgc aag cag atg aac gtg ctg gac agc ttc atc aac tac tac    2880
   gac agc gag aag cac gcc gag aac gcc gtg atc ttc ctg cac ggc aac    2928
   gcc gct agc agc tac ctg tgg agg cac gtg gtg ccc cac atc gag ccc    2976
   gtg gcc agg tgc atc atc ccc gat ctg atc ggc atg ggc aag agc ggc    3024
   aag agc ggc aac ggc agc tac agg ctg ctg gac cac tac aag tac ctg    3072
   acc gcc tgg ttc gag ctc ctg aac ctg ccc aag aag atc atc ttc gtg    3120
   ggc cac gac tgg ggc gcc tgc ctg gcc ttc cac tac agc tac gag cac    3168
   cag gac aag atc aag gcc atc gtg cac gcc gag agc gtg gtg gac gtg    3216
   atc gag agc tgg gac gag tgg cca gac atc gag gag gac atc gcc ctg    3264
   atc aag agc gag gag ggc gag aag atg gtg ctg gag aac aac ttc ttc    3312
   gtg gag acc atg ctg ccc agc aag atc atg aga aag ctg gag ccc gag    3360
   gag ttc gcc gcc tac ctg gag ccc ttc aag gag aag ggc gag gtg aga    3408
   aga ccc acc ctg agc tgg ccc aga gag atc ccc ctg gtg aag ggc ggc     3456
   aag ccc gac gtg gtg cag atc gtg aga aac tac aac gcc tac ctg aga     3504
   gcc agc gac gac ctg ccc aag atg ttc atc gag agc gac ccc ggc ttc     3552
   ttc agc aac gcc atc gtg gag ggc gcc aag aag ttc ccc aac acc gag     3600
   ttc gtg aag gtg aag ggc ctg cac ttc agc cag gag gac gcc ccc gac     3648
   gag atg ggc aag tac atc aag agc ttc gtg gag aga gtg ctg aag aac     3696
   gag cag taa                                                         3705
<210> 6
   <211> 1234
   <212> PRT
   <213> Séquence artificielle
   <223> Description de la séquence artificielle:fusionOBRluc
<400> 6
<210> 7
   <211> 3486
   <212> ADN
   <213> Séquence artificielle
<220>
   <223> Description de la séquence artificielle:fusionOBRyfp
<220>
   <221> CDS
   <222> (1)..(3486)
<400> 7
   atg gtt ctt gcc agc tct acc acc agc atc cac acc atg ctg ctc ctg     48
   Met Val Leu Ala Ser Ser Thr Thr Ser Ile His Thr Met Leu Leu Leu
   1               5                   10                  15
   ctc ctg atg ctc ttc cac ctg gga ctc caa gct tca atc tcg gcg cgc     96
   Leu Leu Met Leu Phe His Leu Gly Leu Gln Ala Ser Ile Ser Ala Arg
               20                  25                  30
   cag gag cag aag ctt atc tcg gag gag gac ctg acg cgt tat cca att     144
   Gln Glu Gln Lys Leu Ile Ser Glu Glu Asp Leu Thr Arg Tyr Pro Ile
           35                  40                  45
   act cct tgg aga ttt aag ttg tct tgc atg cca cca aat tca acc tat     192
   Thr Pro Trp Arg Phe Lys Leu Ser Cys Met Pro Pro Asn Ser Thr Tyr
       50                  55                  60
   gac tac ttc ctt ttg cct gct gga ctc tca aag aat act tca aat tcg     240
   Asp Tyr Phe Leu Leu Pro Ala Gly Leu Ser Lys Asn Thr Ser Asn Ser
   65 70 75 80
   aat gga cat tat gag aca gct gtt gaa cct aag ttt aat tca agt ggt 288
   Asn Gly His Tyr Glu Thr Ala Val Glu Pro Lys Phe Asn Ser Ser Gly
   85 90 95
   act cac ttt tct aac tta tcc aaa aca act ttc cac tgt tgc ttt cgg 336
   Thr His Phe Ser Asn Leu Ser Lys Thr Thr Phe His Cys Cys Phe Arg
   100 105 110
   agt gag caa gat aga aac tgc tcc tta tgt gca gac aac att gaa gga 384
   Ser Glu Gln Asp Arg Asn Cys Ser Leu Cys Ala Asp Asn Ile Glu Gly
   115 120 125
   acg aca ttt gtt tca aca gta aat tct tta gtt ttt caa caa ata gat 432
   Thr Thr Phe Val Ser Thr Val Asn Ser Leu Val Phe Gln Gln Ile Asp
   130 135 140
   gca aac tgg aac ata cag tgc tgg cta aaa gga gac tta aaa tta ttc 480
   Ala Asn Trp Asn Ile Gln Cys Trp Leu Lys Gly Asp Leu Lys Leu Phe
   145 150 155 160
   atc tgt tat gtg gag tca tta ttt aag aat cta ttc agg aat tat aac 528
   Ile Cys Tyr Val Glu Ser Leu Phe Lys Asn Leu Phe Arg Asn Tyr Asn
   165 170 175
   tat aag gtc cat ctt tta tat gtt ctg cct gaa gtg tta gaa gat tca 576
   Tyr Lys Val His Leu Leu Tyr Val Leu Pro Glu Val Leu Glu Asp Ser
   180 185 190
   cct ctg gtt ccc caa aaa ggc agt ttt cag atg gtt cac tgc aat tgc 624
   Pro Leu Val Pro Gln Lys Gly Ser Phe Gln Met Val His Cys Asn Cys
   195 200 205
   agt gtt cat gaa tgt tgt gaa tgt ctt gtg cct gtg cca aca gcc aaa 672
   Ser Val His Glu Cys Cys Glu Cys Leu Val Pro Val Pro Thr Ala Lys
   210 215 220
   ctc aac gac act ctc ctt atg tgt ttg aaa atc aca tct ggt gga gta 720
   Leu Asn Asp Thr Leu Leu Met Cys Leu Lys Ile Thr Ser Gly Gly Val
   225 230 235 240
   att ttc cgg tca cct cta atg tca gtt cag ccc ata aat atg gtg aag 768
   Ile Phe Arg Ser Pro Leu Met Ser Val Gln Pro Ile Asn Met Val Lys
   245 250 255
   cct gat cca cca tta ggt ttg cat atg gaa atc aca gat gat ggt aat 816
   Pro Asp Pro Pro Leu Gly Leu His Met Glu Ile Thr Asp Asp Gly Asn
   260 265 270
   tta aag att tct tgg tcc agc cca cca ttg gta cca ttt cca ctt caa 864
   Leu Lys Ile Ser Trp Ser Ser Pro Pro Leu Val Pro Phe Pro Leu Gln
   275 280 285
   tat caa gtg aaa tat tca gag aat tct aca aca gtt atc aga gaa gct 912
   Tyr Gln Val Lys Tyr Ser Glu Asn Ser Thr Thr Val Ile Arg Glu Ala
   290 295 300
   gac aag att gtc tca gct aca tcc ctg cta gta gac agt ata ctt cct 960
   Asp Lys Ile Val Ser Ala Thr Ser Leu Leu Val Asp Ser Ile Leu Pro
   305 310 315 320
   ggg tct tcg tat gag gtt cag gtg agg ggc aag aga ctg gat ggc cca    1008
   Gly Ser Ser Tyr Glu Val Gln Val Arg Gly Lys Arg Leu Asp Gly Pro
                   325                 330                 335
   gga atc tgg agt gac tgg agt act cct cgt gtc ttt acc aca caa gat    1056
   Gly Ile Trp Ser Asp Trp Ser Thr Pro Arg Val Phe Thr Thr Gln Asp
               340                 345                 350
   gtc ata tac ttt cca cct aaa att ctg aca agt gtt ggg tct aat gtt    1104
   Val Ile Tyr Phe Pro Pro Lys Ile Leu Thr Ser Val Gly Ser Asn Val
           355                 360                 365
   tct ttt cac tgc atc tat aag aag gaa aac aag att gtt ccc tca aaa    1152
   Ser Phe His Cys Ile Tyr Lys Lys Glu Asn Lys Ile Val Pro Ser Lys
       370                 375                 380
   gag att gtt tgg tgg atg aat tta gct gag aaa att cct caa agc cag    1200
   Glu Ile Val Trp Trp Met Asn Leu Ala Glu Lys Ile Pro Gln Ser Gln
   385                 390                 395                 400
   tat gat gtt gtg agt gat cat gtt agc aaa gtt act ttt ttc aat ctg    1248
   Tyr Asp Val Val Ser Asp His Val Ser Lys Val Thr Phe Phe Asn Leu
                   405                 410                 415
   aat gaa acc aaa cct cga gga aag ttt acc tat gat gca gtg tac tgc    1296
   Asn Glu Thr Lys Pro Arg Gly Lys Phe Thr Tyr Asp Ala Val Tyr Cys
               420                 425                 430
   tgc aat gaa cat gaa tgc cat cat cgc tat gct gaa tta tat gtg att    1344
   Cys Asn Glu His Glu Cys His His Arg Tyr Ala Glu Leu Tyr Val Ile
           435                 440                 445
   gat gtc aat atc aat atc tca tgt gaa act gat ggg tac tta act aaa    1392
   Asp Val Asn Ile Asn Ile Ser Cys Glu Thr Asp Gly Tyr Leu Thr Lys
       450                 455                 460
   atg act tgc aga tgg tca acc agt aca atc cag tca ctt gcg gaa agc    1440
   Met Thr Cys Arg Trp Ser Thr Ser Thr Ile Gln Ser Leu Ala Glu Ser
   465                 470                 475                 480
   act ttg caa ttg agg tat cat agg agc agc ctt tac tgt tct gat att    1488
   Thr Leu Gln Leu Arg Tyr His Arg Ser Ser Leu Tyr Cys Ser Asp Ile
                   485                 490                 495
   cca tct att cat ccc ata tct gag ccc aaa gat tgc tat ttg cag agt    1536
   Pro Ser Ile His Pro Ile Ser Glu Pro Lys Asp Cys Tyr Leu Gln Ser
               500                 505                 510
   gat ggt ttt tat gaa tgc att ttc cag cca atc ttc cta tta tct ggc    1584
   Asp Gly Phe Tyr Glu Cys Ile Phe Gln Pro Ile Phe Leu Leu Ser Gly
           515                 520                 525
   tac aca atg tgg att agg atc aat cac tct cta ggt tca ctt gac tct    1632
   Tyr Thr Met Trp Ile Arg Ile Asn His Ser Leu Gly Ser Leu Asp Ser
       530                 535                 540
   cca cca aca tgt gtc ctt cct gat tct gtg gtg aag cca ctg cct cca    1680
   Pro Pro Thr Cys Val Leu Pro Asp Ser Val Val Lys Pro Leu Pro Pro
   545                 550                 555                 560
   tcc agt gtg aaa gca gaa att act ata aac att gga tta ttg aaa ata     1728
   Ser Ser Val Lys Ala Glu Ile Thr Ile Asn Ile Gly Leu Leu Lys Ile
                   565                 570                 575
   tct tgg gaa aag cca gtc ttt cca gag aat aac ctt caa ttc cag att     1776
   Ser Trp Glu Lys Pro Val Phe Pro Glu Asn Asn Leu Gln Phe Gln Ile
               580                 585                 590
   cgc tat ggt tta agt gga aaa gaa gta caa tgg aag atg tat gag gtt     1824
   Arg Tyr Gly Leu Ser Gly Lys Glu Val Gln Trp Lys Met Tyr Glu Val
           595                 600                 605
   tat gat gca aaa tca aaa tct gtc agt ctc cca gtt cca gac ttg tgt     1872
   Tyr Asp Ala Lys Ser Lys Ser Val Ser Leu Pro Val Pro Asp Leu Cys
       610                 615                 620
   gca gtc tat gct gtt cag gtg cgc tgt aag agg cta gat gga ctg gga     1920
   Ala Val Tyr Ala Val Gln Val Arg Cys Lys Arg Leu Asp Gly Leu Gly
   625                 630                 635                 640
   tat tgg agt aat tgg agc aat cca gcc tac aca gtt gtc atg gat ata     1968
   Tyr Trp Ser Asn Trp Ser Asn Pro Ala Tyr Thr Val Val Met Asp Ile
                   645                 650                 655
   aaa gtt cct atg aga gga cct gaa ttt tgg aga ata att aat gga gat     2016
   Lys Val Pro Met Arg Gly Pro Glu Phe Trp Arg Ile Ile Asn Gly Asp
               660                 665                 670
   act atg aaa aag gag aaa aat gtc act tta ctt tgg aag ccc ctg atg     2064
   Thr Met Lys Lys Glu Lys Asn Val Thr Leu Leu Trp Lys Pro Leu Met
           675                 680                 685
   aaa aat gac tca ttg tgc agt gtt cag aga tat gtg ata aac cat cat     2112
   Lys Asn Asp Ser Leu Cys Ser Val Gln Arg Tyr Val Ile Asn His His
       690                 695                 700
   act tcc tgc aat gga aca tgg tca gaa gat gtg gga aat cac acg aaa     2160
   Thr Ser Cys Asn Gly Thr Trp Ser Glu Asp Val Gly Asn His Thr Lys
   705                 710                 715                 720
   ttc act ttc ctg tgg aca gag caa gca cat act gtt acg gtt ctg gcc     2208
   Phe Thr Phe Leu Trp Thr Glu Gln Ala His Thr Val Thr Val Leu Ala
                   725                 730                 735
   atc aat tca att ggt gct tct gtt gca aat ttt aat tta acc ttt tca     2256
   Ile Asn Ser Ile Gly Ala Ser Val Ala Asn Phe Asn Leu Thr Phe Ser
               740                 745                 750
   tgg cct atg agc aaa gta aat atc gtg cag tca ctc agt gct tat cct     2304
   Trp Pro Met Ser Lys Val Asn Ile Val Gln Ser Leu Ser Ala Tyr Pro
           755                 760                 765
   tta aac agc agt tgt gtg att gtt tcc tgg ata cta tca ccc agt gat     2352
   Leu Asn Ser Ser Cys Val Ile Val Ser Trp Ile Leu Ser Pro Ser Asp
       770                 775                 780
   tac aag cta atg tat ttt att att gag tgg aaa aat ctt aat gaa gat     2400
   Tyr Lys Leu Met Tyr Phe Ile Ile Glu Trp Lys Asn Leu Asn Glu Asp
   785                 790                 795                 800
   ggt gaa ata aaa tgg ctt aga atc tct tca tct gtt aag aag tat tat     2448
   Gly Glu Ile Lys Trp Leu Arg Ile Ser Ser Ser Val Lys Lys Tyr Tyr
                   805                     810                 815
   atc cat gat cat ttt atc ccc att gag aag tac cag ttc agt ctt tac    2496
   Ile His Asp His Phe Ile Pro Ile Glu Lys Tyr Gln Phe Ser Leu Tyr
               820                 825                 830
   cca ata ttt atg gaa gga gtg gga aaa cca aag ata att aat agt ttc    2544
   Pro Ile Phe Met Glu Gly Val Gly Lys Pro Lys Ile Ile Asn Ser Phe
           835                 840                 845
   act caa gat gat att gaa aaa cac cag agt gat gca ggt tta tat gta    2592
   Thr Gln Asp Asp Ile Glu Lys His Gln Ser Asp Ala Gly Leu Tyr Val
       850                 855                 860
   att gtg cca gta att att tcc tct tcc atc tta ttg ctt gga aca tta    2640
   Ile Val Pro Val Ile Ile Ser Ser Ser Ile Leu Leu Leu Gly Thr Leu
   865                 870                 875                 880
   tta ata tca cac caa aga atg aaa aag cta ttt tgg gaa gat gtt ccg    2688
   Leu Ile Ser His Gln Arg Met Lys Lys Leu Phe Trp Glu Asp Val Pro
                   885                 890                 895
   aac ccc aag aat tgt tcc tgg gca caa gga ctt aat ttt cag aag aga    2736
   Asn Pro Lys Asn Cys Ser Trp Ala Gln Gly Leu Asn Phe Gln Lys Arg
               900                 905                 910
   acg gac att ctg gat cca ccg gtc gcc acc atg gtg agc aag ggc gag    2784
   Thr Asp Ile Leu Asp Pro Pro Val Ala Thr Met Val Ser Lys Gly Glu
           915                 920                 925
   gag ctg ttc acc ggg gtg gtg ccc atc ctg gtc gag ctg gac ggc gac    2832
   Glu Leu Phe Thr Gly Val Val Pro Ile Leu Val Glu Leu Asp Gly Asp
       930                 935                 940
   gta aac ggc cac aag ttc agc gtg tcc ggc gag ggc gag ggc gat gcc    2880
   Val Asn Gly His Lys Phe Ser Val Ser Gly Glu Gly Glu Gly Asp Ala
   945                 950                 955                 960
   acc tac ggc aag ctg acc ctg aag ttc atc tgc acc acc ggc aag ctg    2928
   Thr Tyr Gly Lys Leu Thr Leu Lys Phe Ile Cys Thr Thr Gly Lys Leu
                   965                 970                 975
   ccc gtg ccc tgg ccc acc ctc gtg acc acc ttc ggc tac ggc gtg cag    2976
   Pro Val Pro Trp Pro Thr Leu Val Thr Thr Phe Gly Tyr Gly Val Gln
               980                 985                 990
   tgc ttc gcc cgc tac ccc gac cac atg cgc cag cac gac ttc ttc aag    3024
   Cys Phe Ala Arg Tyr Pro Asp His Met Arg Gln His Asp Phe Phe Lys
           995                 1000                1005
   tcc gcc atg ccc gaa ggc tac gtc cag gag cgc acc atc ttc ttc aag    3072
   Ser Ala Met Pro Glu Gly Tyr Val Gln Glu Arg Thr Ile Phe Phe Lys
       1010                1015                1020
   gac gac ggc aac tac aag acc cgc gcc gag gtg aag ttc gag ggc gac    3120
   Asp Asp Gly Asn Tyr Lys Thr Arg Ala Glu Val Lys Phe Glu Gly Asp
   1025                1030                1035                1040
   acc ctg gtg aac cgc atc gag ctg aag ggc atc gac ttc aag gag gac    3168
   Thr Leu Val Asn Arg Ile Glu Leu Lys Gly Ile Asp Phe Lys Glu Asp
                       1045                1050                1055
   ggc aac atc ctg ggg cac aag ctg gag tac aac tac aac agc cac aac      3216
   Gly Asn Ile Leu Gly His Lys Leu Glu Tyr Asn Tyr Asn Ser His Asn
                   1060                1065                1070
   gtc tat atc atg gcc gac aag cag aag aac ggc atc aag gtg aac ttc      3264
   Val Tyr Ile Met Ala Asp Lys Gln Lys Asn Gly Ile Lys Val Asn Phe
               1075                1080                1085
   aag atc cgc cac aac atc gag gac ggc agc gtg cag ctc gcc gac cac      3312
   Lys Ile Arg His Asn Ile Glu Asp Gly Ser Val Gln Leu Ala Asp His
           1090                1095                1100
   tac cag cag aac acc ccc atc ggc gac ggc ccc gtg ctg ctg ccc gac      3360
   Tyr Gln Gln Asn Thr Pro Ile Gly Asp Gly Pro Val Leu Leu Pro Asp
   1105                1110                1115                1120
   aac cac tac ctg agc tac cag tcc gcc ctg agc aaa gac ccc aac gag      3408
   Asn His Tyr Leu Ser Tyr Gln Ser Ala Leu Ser Lys Asp Pro Asn Glu
                       1125                1130                1135
   aag cgc gat cac atg gtc ctg ctg gag ttc gtg acc gcc gcc ggg atc      3456
   Lys Arg Asp His Met Val Leu Leu Glu Phe Val Thr Ala Ala Gly Ile
                   1140                1145                1150
   act ctc ggc atg gac gag ctg tac aag taa                              3486
   Thr Leu Gly Met Asp Glu Leu Tyr Lys
               1155                1160
<210> 8
   <211> 1161
   <212> PRT
   <213> Séquence artificielle
   <223> Description de la séquence artificielle:fusionOBRyfp
<400> 8
   Met Val Leu Ala Ser Ser Thr Thr Ser Ile His Thr Met Leu Leu Leu
    1               5                  10                  15
   Leu Leu Met Leu Phe His Leu Gly Leu Gln Ala Ser Ile Ser Ala Arg
               20                  25                  30
   Gln Glu Gln Lys Leu Ile Ser Glu Glu Asp Leu Thr Arg Tyr Pro Ile
           35                  40                  45
   Thr Pro Trp Arg Phe Lys Leu Ser Cys Met Pro Pro Asn Ser Thr Tyr
       50                  55                  60
   Asp Tyr Phe Leu Leu Pro Ala Gly Leu Ser Lys Asn Thr Ser Asn Ser
   65                  70                  75                  80
   Asn Gly His Tyr Glu Thr Ala Val Glu Pro Lys Phe Asn Ser Ser Gly
                   85                  90                  95
   Thr His Phe Ser Asn Leu Ser Lys Thr Thr Phe His Cys Cys Phe Arg
               100                 105                 110
   Ser Glu Gln Asp Arg Asn Cys Ser Leu Cys Ala Asp Asn Ile Glu Gly
   115 120 125
   Thr Thr Phe Val Ser Thr Val Asn Ser Leu Val Phe Gln Gln Ile Asp
   130 135 140
   Ala Asn Trp Asn Ile Gln Cys Trp Leu Lys Gly Asp Leu Lys Leu Phe
   145 150 155 160
   Ile Cys Tyr Val Glu Ser Leu Phe Lys Asn Leu Phe Arg Asn Tyr Asn
   165 170 175
   Tyr Lys Val His Leu Leu Tyr Val Leu Pro Glu Val Leu Glu Asp Ser
   180 185 190
   Pro Leu Val Pro Gln Lys Gly Ser Phe Gln Met Val His Cys Asn Cys
   195 200 205
   Ser Val His Glu Cys Cys Glu Cys Leu Val Pro Val Pro Thr Ala Lys
   210 215 220
   Leu Asn Asp Thr Leu Leu Met Cys Leu Lys Ile Thr Ser Gly Gly Val
   225 230 235 240
   Ile Phe Arg Ser Pro Leu Met Ser Val Gln Pro Ile Asn Met Val Lys
   245 250 255
   Pro Asp Pro Pro Leu Gly Leu His Met Glu Ile Thr Asp Asp Gly Asn
   260 265 270
   Leu Lys Ile Ser Trp Ser Ser Pro Pro Leu Val Pro Phe Pro Leu Gln
   275 280 285
   Tyr Gln Val Lys Tyr Ser Glu Asn Ser Thr Thr Val Ile Arg Glu Ala
   290 295 300
   Asp Lys Ile Val Ser Ala Thr Ser Leu Leu Val Asp Ser Ile Leu Pro
   305 310 315 320
   Gly Ser Ser Tyr Glu Val Gln Val Arg Gly Lys Arg Leu Asp Gly Pro
   325 330 335
   Gly Ile Trp Ser Asp Trp Ser Thr Pro Arg Val Phe Thr Thr Gln Asp
   340 345 350
   Val Ile Tyr Phe Pro Pro Lys Ile Leu Thr Ser Val Gly Ser Asn Val
   355 360 365
   Ser Phe His Cys Ile Tyr Lys Lys Glu Asn Lys Ile Val Pro Ser Lys
   370 375 380
   Glu Ile Val Trp Trp Met Asn Leu Ala Glu Lys Ile Pro Gln Ser Gln
   385 390 395 400
   Tyr Asp Val Val Ser Asp His Val Ser Lys Val Thr Phe Phe Asn Leu
   405 410 415
   Asn Glu Thr Lys Pro Arg Gly Lys Phe Thr Tyr Asp Ala Val Tyr Cys
   420 425 430
   Cys Asn Glu His Glu Cys His His Arg Tyr Ala Glu Leu Tyr Val Ile
   435 440 445
   Asp Val Asn Ile Asn Ile Ser Cys Glu Thr Asp Gly Tyr Leu Thr Lys
   450 455 460
   Met Thr Cys Arg Trp Ser Thr Ser Thr Ile Gln Ser Leu Ala Glu Ser
   465 470 475 480
   Thr Leu Gln Leu Arg Tyr His Arg Ser Ser Leu Tyr Cys Ser Asp Ile
   485 490 495
   Pro Ser Ile His Pro Ile Ser Glu Pro Lys Asp Cys Tyr Leu Gln Ser
   500 505 510
   Asp Gly Phe Tyr Glu Cys Ile Phe Gln Pro Ile Phe Leu Leu Ser Gly
   515 520 525
   Tyr Thr Met Trp Ile Arg Ile Asn His Ser Leu Gly Ser Leu Asp Ser
   530 535 540
   Pro Pro Thr Cys Val Leu Pro Asp Ser Val Val Lys Pro Leu Pro Pro
   545 550 555 560
   Ser Ser Val Lys Ala Glu Ile Thr Ile Asn Ile Gly Leu Leu Lys Ile
   565 570 575
   Ser Trp Glu Lys Pro Val Phe Pro Glu Asn Asn Leu Gln Phe Gln Ile
   580 585 590
   Arg Tyr Gly Leu Ser Gly Lys Glu Val Gln Trp Lys Met Tyr Glu Val
   595 600 605
   Tyr Asp Ala Lys Ser Lys Ser Val Ser Leu Pro Val Pro Asp Leu Cys
   610 615 620
   Ala Val Tyr Ala Val Gln Val Arg Cys Lys Arg Leu Asp Gly Leu Gly
   625 630 635 640
   Tyr Trp Ser Asn Trp Ser Asn Pro Ala Tyr Thr Val Val Met Asp Ile
   645 650 655
   Lys Val Pro Met Arg Gly Pro Glu Phe Trp Arg Ile Ile Asn Gly Asp
   660 665 670
   Thr Met Lys Lys Glu Lys Asn Val Thr Leu Leu Trp Lys Pro Leu Met
   675 680 685
   Lys Asn Asp Ser Leu Cys Ser Val Gln Arg Tyr Val Ile Asn His His
   690 695 700
   Thr Ser Cys Asn Gly Thr Trp Ser Glu Asp Val Gly Asn His Thr Lys
   705 710 715 720
   Phe Thr Phe Leu Trp Thr Glu Gln Ala His Thr Val Thr Val Leu Ala
   725 730 735
   Ile Asn Ser Ile Gly Ala Ser Val Ala Asn Phe Asn Leu Thr Phe Ser
   740 745 750
   Trp Pro Met Ser Lys Val Asn Ile Val Gln Ser Leu Ser Ala Tyr Pro
   755 760 765
   Leu Asn Ser Ser Cys Val Ile Val Ser Trp Ile Leu Ser Pro Ser Asp
       770                 775                 780
   Tyr Lys Leu Met Tyr Phe Ile Ile Glu Trp Lys Asn Leu Asn Glu Asp
   785                 790                 795                 800
   Gly Glu Ile Lys Trp Leu Arg Ile Ser Ser Ser Val Lys Lys Tyr Tyr
                   805                 810                 815
   Ile His Asp His Phe Ile Pro Ile Glu Lys Tyr Gln Phe Ser Leu Tyr
                   820                 825                 830
   Pro Ile Phe Met Glu Gly Val Gly Lys Pro Lys Ile Ile Asn Ser Phe
           835                 840                 845
   Thr Gln Asp Asp Ile Glu Lys His Gln Ser Asp Ala Gly Leu Tyr Val
       850                 855                 860
   Ile Val Pro Val Ile Ile Ser Ser Ser Ile Leu Leu Leu Gly Thr Leu
   865                 870                 875                 880
   Leu Ile Ser His Gln Arg Met Lys Lys Leu Phe Trp Glu Asp Val Pro
                   885                 890                 895
   Asn Pro Lys Asn Cys Ser Trp Ala Gln Gly Leu Asn Phe Gln Lys Arg
               900                 905                 910
   Thr Asp Ile Leu Asp Pro Pro Val Ala Thr Met Val Ser Lys Gly Glu
           915                 920                 925
   Glu Leu Phe Thr Gly Val Val Pro Ile Leu Val Glu Leu Asp Gly Asp
       930                 935                 940
   Val Asn Gly His Lys Phe Ser Val Ser Gly Glu Gly Glu Gly Asp Ala
   945                 950                 955                 960
   Thr Tyr Gly Lys Leu Thr Leu Lys Phe Ile Cys Thr Thr Gly Lys Leu
                   965                 970                 975
   Pro Val Pro Trp Pro Thr Leu Val Thr Thr Phe Gly Tyr Gly Val Gln
               980                 985                 990
   Cys Phe Ala Arg Tyr Pro Asp His Met Arg Gln His Asp Phe Phe Lys
           995                 1000                1005
   Ser Ala Met Pro Glu Gly Tyr Val Gln Glu Arg Thr Ile Phe Phe Lys
       1010                1015                1020
   Asp Asp Gly Asn Tyr Lys Thr Arg Ala Glu Val Lys Phe Glu Gly Asp
   025                 1030                1035                1040
   Thr Leu Val Asn Arg Ile Glu Leu Lys Gly Ile Asp Phe Lys Glu Asp
                   1045                1050                1055
   Gly Asn Ile Leu Gly His Lys Leu Glu Tyr Asn Tyr Asn Ser His Asn
               1060                1065                1070
   Val Tyr Ile Met Ala Asp Lys Gln Lys Asn Gly Ile Lys Val Asn Phe
           1075                1080                1085
   Lys Ile Arg His Asn Ile Glu Asp Gly Ser Val Gln Leu Ala Asp His
   1090 1095 1100
   Tyr Gln Gln Asn Thr Pro Ile Gly Asp Gly Pro Val Leu Leu Pro Asp
   105 1110 1115 1120
   Asn His Tyr Leu Ser Tyr Gln Ser Ala Leu Ser Lys Asp Pro Asn Glu
   1125 1130 1135
   Lys Arg Asp His Met Val Leu Leu Glu Phe Val Thr Ala Ala Gly Ile
   1140 1145 1150
   Thr Leu Gly Met Asp Glu Leu Tyr Lys
   1155 1160

## Description

La présente invention est un procédé de détection de ligands du récepteur de la leptine par mise en oeuvre du transfert d'énergie entre des protéines de fusion composées de récepteurs de la leptine et de protéines donneurs ou accepteurs d'énergie.

Sont décrites également des protéines de fusion utiles pour la mise en oeuvre de ce procédé.

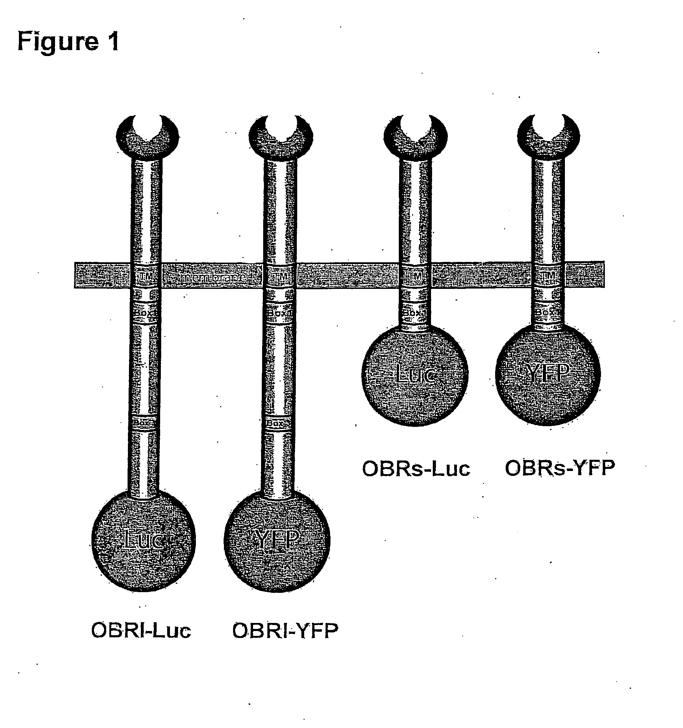

La leptine est une protéine présentant un poids moléculaire de 16 kDa qui est sécrétée par les adipocytes. Cette protéine est associée à la sensation de satiété, et joue un rôle majeur dans le contrôle de la prise de poids, la consommation d'énergie, la formation osseuse, l'angiogénèse mais aussi dans d'autres fonctions physiologiques telles que le déclenchement de la puberté et le contrôle de la reproduction ou la régulation de la réponse immunitaire régulée par les lymphocytes T.
Le récepteur de la leptine (OBR) appartient à la famille des récepteurs aux cytokines. Il est composé, comme l'illustre la figure 1 d'une chaine polypeptidique unique comprenant un domaine transmembranaire (Tartaglia et al., J. Biol. Chem, 272, 6093-6096, 1995). La demande de brevet WO 97/19952 est relative à ce récepteur.
Six isoformes différentes de l'OBR ayant des domaines C-terminaux de longueurs différentes ont été décrits. Ces isoformes dérivent toutes, par épissages alternatifs, d'un gène unique. Il existe également une forme soluble des OBR contenant le site de liaison à la leptine qui correspond au domaine extracellulaire des formes membranaires. Cette forme soluble générée de façon post-traductionelle par protéolyse à la membrane plasmique à partir des formes membranaires se retrouve dans le sang. Une autre forme soluble de l'OBR résultant d'une mutation générant un codon stop avant le domaine transmembranaire est également trouvée dans certaines cas très rares.
Une protéine de fusion constituée de la forme longue du récepteur de la leptine (OBR1) fusionnée à la EGFP (Enhanced Green Fluorescence Protein) a été utilisée par Lundin et al (Biochemica et Biophysica Acta, 1499, 130-138, 2000) pour étudier la localisation du récepteur.
L'activation de l'OBR se ferait par l'intermédiaire d'un complexe tétramèrique composé de deux janus kinase 2 (JAK2) et de deux OBR. L'activation du récepteur induite par la leptine induirait un changement dans la conformation de l'OBR, qui lui même activerait une JAK2, qui à son tour transphosphorylerait une autre JAK2 puis le récepteur OBR.
L'activation de l'OBR apparaît être responsable de tous les effets connus de la leptine tels que la perte de poids et tous les phénomènes impliqués dans les désordres pondéraux.
Les propriétés inhibitrices de la leptine vis à vis de la synthèse osseuse ont ainsi été récemment mises en évidence. La leptine agit en inhibant l'activité des ostéoblastes, une population de cellules responsables de la formation de l'os.
Modifier la leptinémie pourrait permettre de traiter les maladies liées à une diminution de la densité osseuse comme par exemple l'ostéoporose ou à l'inverse celles liées à une calcification importante.
En 1999 Xu et al (Proc Natl Acad Sci U S A 96, 151-156) ont décrit une méthode de détection des interactions protéine-protéine dans des cellules vivantes. Cette méthode a en outre fait l'objet de la demande de brevet WO99/66324.
Cette méthode, appelée BRET (pour Bioluminescent Resonance Energy Transfer ou transfert d'énergie de bioluminescence par résonance), est basée sur un phénomène naturel, l'émission de fluorescence par des organismes marins. La transformation enzymatique, par la luciférase de *Renilla* (Luc), d'un substrat qui peut traverser la membrane génère une bioluminescence, qui à son tour excite un accepteur d'énergie tel que la protéine fluorescente jaune (YFP ou yellow fluorescent protein en anglais). Cette méthode correspond à la LRET (pour Luminescent Resonance Energy Transfer) décrite par Wang et al (Mol Gen Genet 264 : 578-587 (2001)).
L'efficacité du transfert d'énergie dépend de la proximité physique et des orientations respectives de l'accepteur et du donneur. Ainsi la co-expression de la luciférase et de la YFP n'est pas suffisante pour induire un transfert d'énergie car la distance entre les deux partenaires doit être inférieure à 100Å. Afin d'étudier l'interaction entre deux partenaires d'interaction potentiels la première protéine a été fusionnée à la luciférase et la seconde protéine à la YFP. Si les deux protéines interagissent un transfert d'énergie peut être observé.
Depuis, la méthode de BRET a été mise en oeuvre sur un nombre limité de récepteurs, présentant une structure très différente du récepteur de la leptine.
Ainsi quelques auteurs décrivent la mise en oeuvre de la méthode sur des récepteurs de la famille des récepteurs couplés aux protéines G (GPCR) tels que les récepteurs β
2 adrénergiques (Angers et al, 2000, *Proc. Natl. Acad. Sci. U. S. A.* 10.1073), cholesystokines de type A (CCK ;Cheng et al, 2001 *. Biol. Chem.* 276: 48040-48047), et de la thyrotropin releasing hormon (Kroeger et al, 2001, *J. Biol. Chem.* 276: 12736-12743).
Ces récepteurs de taille importante présentent une structure complexe comprenant 7 domaines transmembranaires.
Enfin Boute et al (2001, *Mol Pharmacol* 60: 640-645) ont décrit le suivi de l'activation du récepteur de l'insuline en utilisant le BRET.
Le récepteur de l'insuline est constitué de dimères covalents, et non de complexes non covalents comme le récepteur de la leptine. En outre il comprend une partie intracellulaire assez longue. Enfin les auteurs montrent que le changement de BRET induit par l'insuline ne peut être mis en oeuvre que sur le récepteur solubilisé.

En quelques dizaines d'années, l'obésité est devenue un problème majeur de santé publique dans les pays industrialisés où elle touche maintenant 20 à 30 % de la population. Ces chiffres devraient encore croître de façon alarmante dans les années à venir. Du fait de ses origines multifactorielles qui prennent leurs sources à des degrés plus ou moins importants parmi des facteurs environnementaux d'une part (comportements alimentaires, accès à la nourriture, dépense énergétique,...) et des origines génétiques multiples d'autre part, l'obésité constitue un véritable défi pour la médecine.
De même les maladies de l'os, telles que l'ostéoporose, touchent une partie de plus en plus importante de la population.

La découverte de nouvelles molécules permettant de traiter les diverses maladies liées au récepteur de la leptine, telles que l'obésité et l'ostéoporose, est donc un enjeu majeur de la santé publique.
Il n'existe cependant pas de procédé de criblage spécifique d'agonistes ou d'antagonistes du récepteur de la leptine pouvant être mis en oeuvre à haut flux.

Les demandeurs se sont donc attachés à la mise en oeuvre d'un test de criblage rapide, spécifique et efficace d'agonistes ou d'antagonistes du récepteur de la leptine.

Ils ont montré de manière surprenante que le changement de BRET induit par la leptine pouvait être mis en oeuvre sur l'un des isoformes du récepteur de la leptine, mais qu'il ne pouvait être mis en oeuvre avec tous les isoformes.

Ils ont en outre montré que la mise en oeuvre du BRET sur le récepteur de la leptine est optimale dans certaines conditions opératoires.

La présente invention est donc un procédé de détection de ligands du récepteur de la leptine par mise en oeuvre du transfert d'énergie de résonance entre une première protéine de fusion composée d'un récepteur de la leptine, ou d'une partie substantielle d'un récepteur de la leptine, et d'une protéine donneur d'énergie, ou d'une partie substantielle et active d'une protéine donneur d'énergie, et une seconde protéine de fusion composée d'un récepteur de la leptine, ou d'une partie substantielle d'un récepteur de la leptine, et d'une protéine accepteur d'énergie, ou d'une partie substantielle et active d'une protéine accepteur d'énergie.

Elle est en outre relative à des protéines de fusion pour la mise en oeuvre de ce procédé, ainsi qu'à des acides nucléiques codant ces protéines.

Elle envisage également un procédé de traitement curatif ou préventif de maladies liées à la leptine consistant à administrer un ligand sélectionné par le procédé défini ci-dessus à un patient atteint par la dite maladie.

Un premier objet décrit dans le brevet est donc une protéine de fusion caractérisée en ce qu'elle est composée d'un récepteur de la leptine, ou d'une partie substantielle d'un récepteur de la leptine et d'une protéine accepteur ou donneur d'énergie, ou d'une partie substantielle et active d'une protéine accepteur ou donneur d'énergie.

Les protéines de fusion selon la présente invention sont composées en substance d'une partie correspondant à une partie ou la totalité de la séquence d'un récepteur de la leptine et d'une partie correspondant à une protéine donneur ou accepteur d'énergie. Elles peuvent néanmoins comprendre d'autres séquences d'acides aminés, issues d'autres protéines, telles que des séquences signal. Ainsi la séquence SEQ ID N°4 est constituée d'une partie de la séquence SEQ ID N°2 et d'autres séquences, et en particulier de la séquence signal de l'interleukine 3 de souris.

De manière avantageuse la protéine donneur d'énergie est la luciférase de Renilla. Elle peut néanmoins être toute autre protéine donneur d'énergie telle que le spectre d'émission du donneur chevauche suffisamment le spectre d'excitation de l'accepteur pour permettre un transfert d'énergie efficace entre les deux partenaires. Elle peut ainsi être la GFP, si le transfert d'énergie est le FRET, ou encore l'aequorine si le transfert d'énergie est le CRET. L'aequorine peut être obtenue et utilisée comme décrit dans la demande de brevet EP0 187 519, ou dans l'article de Inouye et al. (PNAS USA 82 : 3154-3158 (1985)).

La protéine fluorescente accepteur d'énergie est quant à elle préférentiellement la DsRed, la GFP ou un mutant de cette protéine, tel que la YFP, l'EYFP, la GFP sauvage, la GFPS65T, ou la Topaz.
Elle peut néanmoins être toute autre protéine fluorescente accepteur d'énergie telle que le spectre d'excitation de l'accepteur et le spectre d'émission du donneur se chevauchent suffisamment pour permettre un transfert d'énergie efficace entre les deux partenaires.

Ces protéines sont connues de l'homme du métier qui peut trouver leurs séquences dans la littérature, notamment dans la revue de Blinks et al. (Pharmacol. Rev. 28 : 1-93 (1976)). En particulier la GFP est décrite par Tsien (Annu. Rev. Biochem. 67 : 509-544 (1998)) et son clonage par Prasher et al. (Gene 111 : 229-233 (1992)). Le clonage de la DsRed est quant à lui décrit par Matz et al. (Nat. Biotechnol. 17 : 969-973 (1999)). Pour la Rluc, l'homme du métier peut se référer à Blinks et al. (Pharmacol. Rev. 28 : 1-93 (1976)) ou encore à Lorenz et al. (PNAS 88: 4438-4442 (1991)).

L'isoforme du récepteur de la leptine, compris en totalité ou en partie dans la protéine de fusion, est une isoforme courte, ou présentant un domaine intracellulaire court.
Une telle isoforme comprend avantageusement un domaine intracellulaire Box1, mais ne comprend pas de domaine intracellulaire Box 3.

De manière préférentielle cette isoforme est l'isoforme OBRs et encore plus préférentiellement l'isoforme OBRs humain. Cette isoforme peut néanmoins être originaire de toute autre espèce.

Elle peut aussi être toute autre isoforme courte, préférentiellement comprenant au moins le domaine extracellulaire de l'OBR, telle la forme soluble de l'OBR contenant le site de liaison à la leptine décrite par Lee et al (Nature 379, 632-635 (1996)), Gavrilova et al (JBC 272 :30546-30551 (1997)) Maamr. et al. (Endocrinology 142 :4389-4393 (2001)) ou Clement et al. (Nature 392 :398-401 (1998)).

Selon un mode de mise en oeuvre particulièrement préférentiel l'isoforme est l'isoforme OBRs humaine de séquence SEQ ID °2. Elle peut aussi être un variant de cette protéine présentant une identité d'au moins 65%, préférentiellement d'au moins 75%, et encore plus préférentiellement d'au moins 85% ou 95%, avec la séquence SEQ ID °2.

La protéine de fusion peut ne comprendre qu'une partie de l'isoforme OBRs humaine. Avantageusement elle comprend la partie comprise entre les acides aminés 46 et 866 de la séquence SEQ ID°2.
La partie correspondant au récepteur à la leptine peut ainsi présenter la séquence SEQ ID°4 ou un variant de cette séquence présentant une identité d'au moins 65%, préférentiellement d'au moins 75%, et encore plus préférentiellement d'au moins 85% ou 95%.

De manière particulièrement avantageuse la protéine de fusion donneur présente la séquence SEQ ID N°6 ou un variant de cette séquence présentant une identité d'au moins 65%.

De manière particulièrement avantageuse la protéine de fusion accepteur présente la séquence SEQ ID N°8 ou un variant de cette séquence présentant une identité d'au moins 65%.

D'autres objets décrits dans le brevet sont des acides nucléiques codant pour ces protéines. De tels acides nucléiques peuvent être des ADN complémentaires ou génomiques, ou des ARN. Ces acides nucléiques ou polynucléotides peuvent être sous forme simple chaîne ou sous la forme de duplex.

Ils sont de manière particulièrement avantageuse des ADN complémentaires.

De manière préférentielle l'acide nucléique a une identité d'au moins 65%, préférentiellement d'au moins 75%, et encore plus préférentiellement d'au moins 85% ou 95%, d'identité en nucléotides avec un acide nucléique de séquence SEQ ID N°5 ou SEQ ID N°7.

Selon encore un autre aspect, l'acide nucléique hybride, dans des conditions d'hybridation de forte stringence, avec un acide nucléique tel que défini ci-avant, et plus particulièrement un acide nucléique de séquences nucléotidiques SEQ ID N°5 et SEQ ID N°7, ou un acide nucléique de séquence complémentaire.

Le «pourcentage d'identité » entre deux séquences de nucléotides ou d'acides aminés, au sens de la présente invention, peut être déterminé en comparant deux séquences alignées de manière optimale, à travers une fenêtre de comparaison.

La partie de la séquence nucléotidique ou polypeptidique dans la fenêtre de comparaison peut ainsi comprendre des additions ou des délétions (par exemple des « gaps ») par rapport à la séquence de référence (qui ne comprend pas ces additions ou ces délétions) de manière à obtenir un alignement optimal des deux séquences.

Le pourcentage est calculé en déterminant le nombre de positions auxquelles une base nucléique ou un résidu d'aminoacide identique est observé pour les deux séquences (nucléique ou peptidique) comparées, puis en divisant le nombre de positions auquel il y a identité entre les deux bases ou résidus d'aminoacides par le nombre total de positions dans la fenêtre de comparaison, puis en multipliant le résultat par 100 afin d'obtenir le pourcentage d'identité de séquence.

L'alignement optimal des séquences pour la comparaison peut être réalisé de manière informatique à l'aide d'algorithmes connus contenus dans le package de la Société WISCONSIN GENETICS SOFTWARE PACKAGE, GENETICS COMPUTER GROUP (GCG), 575 Science Doctor , Madison, WISCONSIN.

A titre d'illustration, le pourcentage d'identité de séquence pourra être effectué à l'aide du logiciel BLAST (versions BLAST 1.4.9 de mars 1996, BLAST 2.0.4 de février 1998 et BLAST 2.0.6 de septembre 1998), en utilisant exclusivement les paramètres par défaut (S. F Altschul et al, J. Mol. Biol. 1990 215 : 403-410, S. F Altschul et al, Nucleic Acids Res. 1997 25 : 3389-3402). Blast recherche des séquences similaires/homologues à une séquence « requête » de référence, à l'aide de l'algorithme d'Altschul et al. La séquence requête et les bases de données utilisées peuvent être peptidiques ou nucléiques, toute combinaison étant possible.

Par « conditions d'hybridation de forte stringence » au sens de la présente invention, on entendra les conditions suivantes :

### 1- Compétition des membranes et PRE HYBRIDATION :

- Mélanger: 40µl ADN sperme de saumon (10mg/ml)+ 40 µl ADN placentaire humain (10mg/ml)
- Dénaturer 5 mn à 96°C, puis plonger dans la glace le mélange.
- Oter le SSC 2X et verser 4 ml de mix formamide dans le tube d'hybridation contenant les membranes.
- Ajouter le mélange des deux ADNs dénaturés.
- Incubation à 42°C pendant 5 à 6 heures, avec rotation.

### 2- Compétition de la sonde marquée :

- Ajouter à la sonde marquée et purifiée 10 à 50 µl ADN Cot I, selon la quantité de répétitions.
- Dénaturer 7 à 10 mn à 95°C.
- Incuber à 65°C pendant 2 à 5 heures.

### 3- Hybridation :

- Oter le mix de pré hybridation.
- Mélanger 40 µl ADN sperme de saumon + 40 µl ADN placentaire humain dénaturer 5 mn à 96°C, puis plonger dans la glace.
- Ajouter dans le tube d'hybridation 4 ml de mix formamide, le mélange des deux ADN et la sonde marquée/ADN Cot I dénaturée.
- Incuber 15 à 20 heures à 42°C, avec rotation.

### 4- Lavages :

- Un lavage à température ambiante dans du SSC 2X, pour rincer.
- 2 fois 5 minutes à température ambiante SSC 2X et SDS 0,1% à 65°C.
- 2 fois 15 minutes à 65°C SSC 1X et SDS 0,1% à 65°C.
Envelopper les membranes dans du Saran et exposer.

Les conditions d'hybridation décrites plus haut sont adaptées à l'hybridation dans des conditions de forte stringence, d'une molécule d'acide nucléique d'une longueur variable de 20 nucléotides à plusieurs centaines de nucléotides.

Il va sans dire que les conditions d'hybridation ci-dessus décrites peuvent être adaptées en fonction de la longueur de l'acide nucléique dont l'hybridation est recherchée ou du type de marquage choisi, selon des techniques connues de l'homme du métier.
Les conditions convenables d'hybridation peuvent par exemple être adaptées selon l'enseignement contenu dans l'ouvrage de HAMES et HIGGINS (1985,"Nucleic acid hybridization : a practical approach", Hames and Higgins Ed., IRL Press, Oxford) ou encore dans l'ouvrage de F.AUSUBEL et al (1989, Current Protocols in Molecular Biology, Green Publishing Associates and Wiley Interscience, N.Y).

Les protéines utiles pour mettre en oeuvre la présente invention peuvent être obtenues par tous moyens connus de l'homme du métier. Elles sont néanmoins avantageusement obtenues par expression des acides nucléiques tels que décrits ci-dessus, codant pour ces protéines, éventuellement insérés dans des vecteurs d'expression, dans des cellules avantageusement choisies, suivie éventuellement par une extraction et une purification qui peut être totale ou partielle.

L'invention est également relative à un vecteur recombinant comprenant un acide nucléique selon l'invention.

Avantageusement, un tel vecteur recombinant comprendra un acide nucléique choisi parmi les acides nucléiques suivants :
a) un acide nucléique codant pour une protéine ayant au moins 65% d'identité en acides aminés avec une séquence SEQ ID N°6 ou SEQ ID N°8 ou un fragment peptidique ou un variant de cette dernière ;
b) un acide nucléique comprenant un polynucléotide ayant une séquence SEQ ID N°5 ou SEQ ID N°7, ou un fragment ou un variant de ce dernier ;
c) un acide nucléique ayant au moins 65% d'identité en nucléotides avec un acide nucléique ayant une séquence SEQ ID N°5 ou SEQ ID N°7 ou un fragment ou un variant de ce dernier ;
d) un acide nucléique hybridant, dans des conditions d'hybridation de forte stringence, avec un acide nucléique de séquences SEQ ID N°5 ou SEQ ID N°7, ou un fragment ou un variant de ce dernier.

Par « vecteur » au sens de la présente invention on entendra une molécule d'ADN ou d'ARN circulaire ou linéaire qui est indifféremment sous forme de simple brin ou double brin.

Selon un mode de réalisation, le vecteur d'expression comprend, outre un acide nucléique conforme à l'invention, des séquences régulatrices permettant d'en diriger la transcription et/ou la traduction.

Selon un mode de réalisation avantageux, un vecteur recombinant utile dans une méthode selon l'invention comprendra notamment les éléments suivants :
(1) des éléments de régulation de l'expression de l'acide nucléique à insérer, tels que des promoteurs et des enhanceurs ;
(2) la séquence codante comprise dans l'acide nucléique conforme à l'invention à insérer dans un tel vecteur, ladite séquence codante étant placée en phase avec les signaux de régulation décrits aux (1) ; et
(3) des séquences d'initiation et d'arrêt de la transcription appropriées.

En outre, les vecteurs recombinants pourront inclure une ou plusieurs origines de réplication chez les hôtes cellulaires dans lesquels leur amplification ou leur expression est recherchée, des marqueurs ou des marqueurs de sélection.

A titre d'exemples, les promoteurs pour cellules eucaryotes comprendront le promoteur de la thymidine kinase du virus HSV ou encore le promoteur de la métallothionéine-L de souris.

De manière générale, pour le choix d'un promoteur adapté, l'homme du métier pourra avantageusement se référer à l'ouvrage de SAMBROOK et al. (1989, "Molecular Cloning : A Laboratory Manual, " 2^{nd} ed., Cold Spring Harbor Laboratory Press, Cold Spring Harbor, NY.) ou encore aux techniques décrites par FULLER et al. (1996, *Immunology in Current Protocols in Molecular Biology,* Ausubel et al).

Les vecteurs préférés selon l'invention sont des plasmides, tels que par exemple les vecteurs pCDNA3 (Invitrogen), pQE70, pQE60, pQE9 (Qiagen), psiX174, pBluescript SA, pNH8A, pNH16A, pNH18A, pNH46A, pWLNEO, pSV2CAT, pOG44, pXTI, pSG(Stratagene).

Il peut s'agir également de vecteurs de type *baculovirus* tel que le vecteur pVL1392/1393 (Pharmingen) utilisé pour transfecter les cellules de la lignée Sf9 (ATCC N°CRL 1711) dérivées de *Spodoptera frugiperda.*

Il peut encore s'agir de vecteurs adénoviraux tels que l'adénovirus humain de type 2 ou 5.

Un vecteur recombinant peut aussi être un vecteur rétroviral ou encore un vecteur adéno-associé (AAV). De tels vecteurs adéno-associés sont par exemple décrits par FLOTTE et al. (1992, *Am. J. Respir. Cell Mol. Biol.,* **7** : 349-356

Le brevet décrit en outre des cellules comprenant une protéine, un acide nucléique ou un vecteur tels que décrits ci dessus, ou des fragments de ces cellules, des lysats de ces cellules ou encore des membranes de ces cellules.

De telles cellules peuvent être cellules isolées d'un organisme et cultivées dans un milieu de croissance adéquat. Elles sont néanmoins préférentiellement des lignées cellulaires. Ainsi de telles lignées sont de manière particulièrement avantageuse les lignées cellulaires HEK 293, COS (ATCC N°CRL 1650), COS-M6 et HeLa (ATCC N°CCL2), ou encore Cv 1 (ATCC N°CCL70), Sf-9 (ATCC N°CRL 1711), CHO (ATCC N°CCL-61) ou 3T3 (ATCC N°CRL-6361).

Les membranes de ces cellules peuvent être préparées par toute méthode connue de l'homme du métier.
Préférentiellement elles seront préparées par broyage mécanique des cellules puis centrifugation des suspensions obtenues, comme illustré dans les exemples qui suivent.

La présente invention est en outre relative à des compostions comprenant des cellules telles que décrites ci dessus et de la saponine.

La présente invention est un procédé de détermination de la liaison de composés au récepteur de la leptine comprenant les étapes consistant à :
- mettre en contact ledit composé avec une protéine de fusion donneur d'énergie telle que décrite ci dessus, et une protéine de fusion accepteur d'énergie telle que décrite ci dessus, ou des cellules, ou des fragments, ou des lysats, ou des membranes de cellules comprenant une telle protéine, et un substrat enzymatique adéquat, et
- à mesurer le transfert d'énergie.

Préférentiellement ledit procédé est mis en oeuvre avec des cellules traitées par de la saponine.
Les protéines de fusion donneur d'énergie et les protéines de fusion accepteur d'énergie sont choisies de manière à ce que l'énergie résultant de l'activation du donneur puisse être transférée de manière efficace à l'accepteur.

Dans un mode de mise en oeuvre avantageux dudit procédé, la protéine de fusion donneur d'énergie est une protéine de fusion entre le récepteur de la leptine, ou une partie substantielle du récepteur de la leptine et la luciférase ou une partie substantielle de la luciférase, auquel cas le substrat est avantageusement la coelenterazine.

Dans un mode de mise en oeuvre préférentiel dudit procédé, la protéine de fusion accepteur d'énergie est une protéine de fusion entre le récepteur de la leptine, ou une partie substantielle du récepteur de la leptine et la YFP ou une partie substantielle de la YFP.

Dans un mode de mise en oeuvre avantageux dudit procédé, le transfert d'énergie mesuré en présence du composé à tester est comparé à celui mesuré en absence du composé à tester.

Préférentiellement le procédé est mis en oeuvre sur des membranes des cellules, telles que décrites ci dessus.

De manière préférentielle les protéines donneur et accepteur selon la présente invention sont choisies afin que le transfert d'énergie se fasse par résonance BRET, ou LRET. Néanmoins un tel transfert d'énergie peut être effectué par FRET (pour Fluorescent Résonance Energy Transfer ou transfert d'énergie de fluorescence par résonance) ou encore par CRET (pour Chemioluminescent Résonance Energy Transfer ou transfert d'énergie de chimioluminescence par résonance).
Quelque soit le type de transfert d'énergie les couples protéine de fusion donneur/protéine de fusion accepteur d'énergie sont choisis afin de permettre un tel transfert.

Le CRET consiste en un transfert d'énergie entre l'aequorine, qui est une luciférase, et la GFP.
Le FRET consiste en un transfert d'énergie entre deux protéines de la famille des GFP ayant des spectres différents.
Pour la mise en oeuvre de ces transferts l'homme du métier peut se référer à Baubet et al. (PNAS USA 97 : 7260-7265 (2000)) pour la CRET, à Matyus (J Photochem Photobiol B 12: 323-337 (1992)) et Pollok et Heim (Trends Cell Biol 9:57-60 (1999)) pour la FRET.

Un autre objet de la présente invention est un procédé de criblage ou de détection de composés destinés à la prévention et / ou au traitement de pathologies liées à la leptine comprenant les étapes consistant à :
- mettre en contact ledit composé avec une protéine de fusion donneur d'énergie telle que décrite ci dessus, et une protéine de fusion accepteur d'énergie telle que décrite ci dessus, ou des cellules en absence ou présence de saponine, ou des fragments, ou des lysats, ou des membranes de cellules comprenant de telles protéines, et éventuellement un substrat enzymatique adéquat, et
- à mesurer le transfert d'énergie.

Un tel procédé peut être utilisé pour le criblage d'agonistes ou d'antagonistes du récepteur de la leptine.

Le procédé selon la présente invention est compatible avec les plaques à 96 ou 384 puits généralement utilisées. Il ne nécessite pas l'utilisation de molécules radioactives, est sensible, reproductible, rapide et le résultat est facile à lire. En effet ce procédé a un bon rapport signal/bruit de fonds et une faible réactivité croisée avec d'autres ligands que la leptine. Ceci s'explique au moins en partie par le fait que la détection de l'activation de l'OBR est directement effectuée au niveau du récepteur, ce qui permet d'éliminer des sources possibles de réactivité croisée à d'autres niveaux des voies de signalisation comme on peut l'observer dans le cas de dosages basés sur des gènes rapporteur ou sur la croissance de cellules.
De plus ce procédé n'est pas limité à une voie de transduction ayant un signal spécifique mais au contraire est susceptible de détecter toutes les molécules interagissant avec les OBR.
Cette caractéristique est particulièrement intéressante pour la mise en oeuvre de criblage à grande échelle, puisque de plus en plus de ligands de récepteurs membranaires s'avèrent activer certaines voies mais pas d'autres voies.

La présente invention est en outre relative à l'utilisation de composés sélectionnés par un procédé consistant à :
- mettre en contact ledit composé avec une protéine de fusion donneur d'énergie, et une protéine de fusion accepteur d'énergie telle que décrite ci-dessus, ou des cellules, ou des fragments, ou des lysats, ou des membranes de cellules comprenant une telle protéine, et éventuellement un substrat enzymatique adéquat, et
- à mesurer le transfert d'énergie, pour la fabrication d'un médicament pour le traitement curatif ou préventif de maladies liées à la leptine ou à son récepteur.

Elle est enfin relative à un procédé de traitement curatif ou préventif de maladies liées à la leptine ou à son récepteur comprenant les étapes de:
- sélection dudit composé par un procédé consistant à :
   +mettre en contact ledit composé avec une protéine de fusion donneur d'énergie et une protéine de fusion accepteur d'énergie, ou des cellules, ou des fragments, des lysats, ou des membranes de cellules comprenant une telle protéine, et un substrat enzymatique adéquat, et
   +à mesurer le transfert d'énergie, et
- d'administration dudit composé à un patient atteint par la dite maladie.

De telles maladies peuvent être des maladies liées à une diminution de la densité osseuse comme par exemple l'ostéoporose ou à l'inverse celles liées à une calcification importante.
Elles peuvent aussi être des maladies ayant un effet sur le poids, telles que l'obésité, le diabète ou l'anorexie.
Les composés identifiés par l'une des méthodes de l'invention peuvent être formulés dans des compositions pharmaceutiques en vue d'une administration par voie topique, orale, parentérale, intranasale, intraveineuse, intramusculaire, sous-cutanée, intraoculaire, etc. Préférentiellement, les compositions pharmaceutiques contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Enfin le procédé selon la présente invention permet aussi le criblage de sérum de patients obèses pour la présence ou l'absence de leptine non fonctionnelle ou encore le criblage de molécules interférant avec la dimèrisation de l'OBR.

La figure 1 représente schématiquement les protéines de fusion. Box1 représente le site de liaison de JAK2; Box3 représente le site de liaison des protéines STAT; TM représente le domaine trans-membranaire.

Les figures 2a et 2b illustrent l'expression des constructions OBR dans des cellules COS estimée par des expériences de radio-marquage utilisant la ¹²⁵I-leptine comme radio-ligand. Dans les figures 2a et 2b le contenu cellulaire total en OBR et le pourcentage de sites de liaison à la surface des cellules sont respectivement mesurés.
La figure 2c illustre la localisation cellulaire de l'expression des constructions OBR1-YFP et OBRs-YFP en présence et en absence de leptine.
La figure 2d illustre l'activation de JAK2 par différentes constructions OBR.
La figure 2e illustre l'effet de la stimulation par la leptine de cellules co-exprimant le gène rapporteur pour STAT3 et différentes constructions OBR.

La figure 3 illustre la dimérisation constitutive de OBR. Des cellules HEK 293 exprimant les différentes constructions OBR indiquées sont incubées en présence de coelenterazine. Le transfert d'énergie est mesurée à l'aide d'un luminomètre.

Les figures 4a et 4b illustrent l'effet de la liaison de la leptine sur le BRET constitutif des OBR.

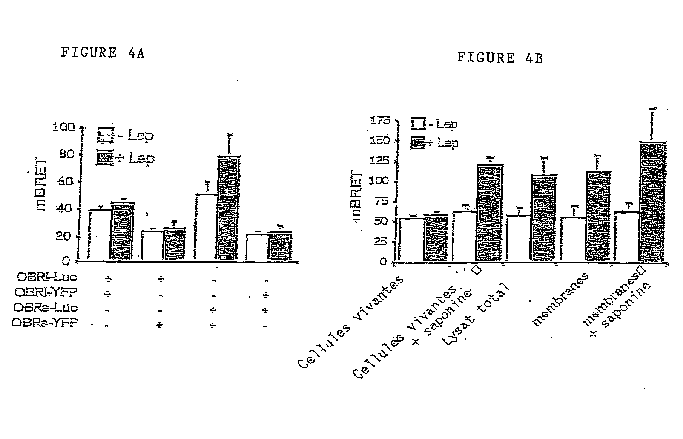

Figure 4a: des cellules HeLa exprimant les différentes constructions OBR indiqués sont incubées en présence de leptine avant d'initier la réaction de la luciférase. Le transfert d'énergie est mesurée à l'aide d'un luminomètre.
Figure 4b: L'effet de la leptine est comparé dans des cellules entières co-exprimant OBRs-Luc et OBR-YFP, en absence ou présence de saponine, dans des lysats totaux et dans des préparations membranaires.

Les figures 5a à 5e illustrent l'optimisation et la caractérisation du changement du BRET induit par la leptine sur les OBRs. Des membranes préparées à partir de cellules HeLa ou COS co-exprimant OBRs-Luc et OBRs-YFP ont été pré-incubées avec ou sans leptine avant d'initier la réaction de la luciférase.
Figure 5a: Optimisation des niveaux d'expression de OBRs-Luc et de OBRs-YFP relatifs et absolus.

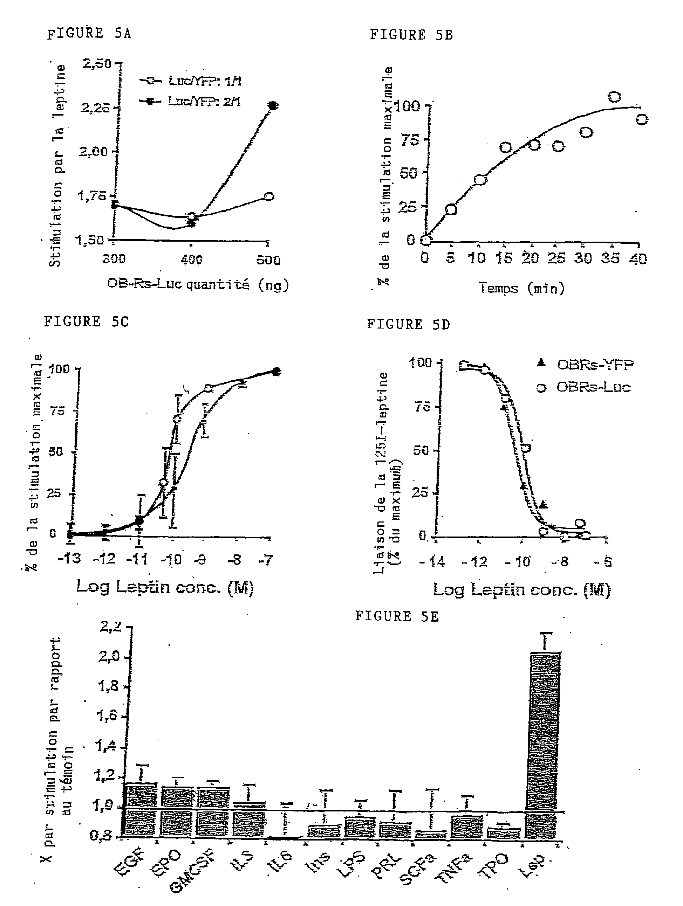

Figure 5b : Variation du signal de BRET induit par la leptine en fonction du temps.
Figure 5c : Courbes dose-réponse BRET/concentration en leptine sur membrane et cellules intacte en présence de saponine (0.05%).
Figure 5d : Compétition de la liaison de la ¹²⁵I-leptine par augmentation des concentrations croissantes de la leptine.
Figure 5e: Spécificité des changements de BRET induits par la leptine. Les membranes ont été pré-incubées avec des concentrations saturantes d'érythropoiétine (EPO, 10U /ml), de trombopoiétine (TPO, 10 nM), de granulocyte macrophage colony stimulating factor (GM-CSF, 250 ng/ml), d'interleukine 3 (IL3, 280 ng/ml), d'interleukine 6 (IL6, 100 ng/ml), de prolactine (PRL, 200 ng/ml), de stem cell factor α (SCFα, 250 ng/ml), d'epidermal growth factor (EGF, 100 ng/ml), d'insuline (Ins, 100 nM), de lipopolysaccharide (LPS, 100 ng/ml) et de tumor necrosis factor α (TNFα, 50ng/ml).

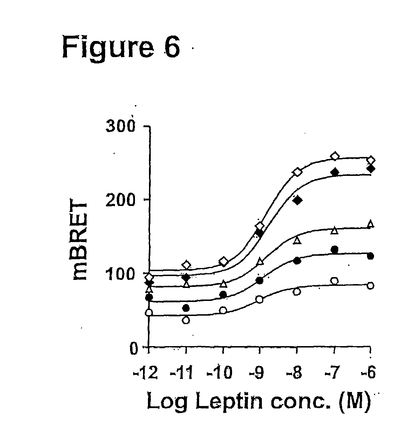

Figure 6 : Co-transfectées de cellules COS avec une quantité constante d'OB-Rs-Luc (50ng) et une quantité croissante d'OB-Rs-YFP : o, 200 ng ; •, 400 ng ; Δ, 800 ◆, 1600 ; 0, 3200. Les mesures de BRET ont été faites sur les cellules en présence de saponine (0,015%), incubées ou non avec des doses croissantes de leptine, et sont exprimées en mBRET.

La présente invention est illustrée sans pour autant être limitée par les exemples qui suivent.

### Matériels et Méthodes utilisés dans les exemples

### Constructions plasmidiques, transfections et culture cellulaire.

Les protéines de fusion OB-R-YFP et OB-R-Luc ont été construites par ligation de la YFP et de Luc à l'extrémité C-terminale des récepteurs par des techniques classiques de biologie moléculaire. Les régions codantes de YFP obtenues à partir du vecteur pGFPtpz-N1 Cytogem® -Topaze (Packard, Meriden, CT) ont été insérées dans le site EcoRV de pcDNA3/CMV (Invitrogen, Groningen, Netherlands) qui contient un site polylinker modifié. La région codante de *Renilla* luciferase a été obtenue à partir de pRL-CMV (Promega, Madison, WI) et insérée dans le site EcoRV du pcDNA3modifié. Les régions codantes de OBR1 et OBRs (don de Dr. Gainsford, Royal Melbourne Hospital, Victoria, Australia) ont été insérées dans les deux vecteurs décrits ci dessus, respectivement dans les sites de clonage EcoR1/BamH1 et Nhel. Les codons Stop ont été délétés par mutagenèse dirigée et la phase de la protéine de fusion a été ajustée.
Les lignées cellulaires HEK 293, COS-M6 et HeLa ont été cultivées dans du DMEM complété avec les composants suivants : 10% (v/v) FBS, 4.5 g/litre glucose, 100 U/ml pénicilline, 0.1 mg/ml streptomycine, 1 mM glutamine (Life Technologies, Gaithersburg, MD).
Les transfections transitoires ont été effectuées en utilisant le réactif de transfection FuGene 6 (Roche, Bale, Suisse).

### Microscopie par Fluorescence.

Deux jours après la transfection avec les constructions YFP, les cellule ont été incubées avec 100 nM leptine durant 60 min et 0.01 mM bis-benzamidine durant 15 min avant d'être lavées dans du PBS et fixées durant 20 min à température ambiante dans une solution froide de 4% paraformaldéhyde dans du PBS. Les coupes sont observées par microscopie fluorescente en utilisant des filtres FITC et DAPI.

### Préparation des membranes et solubilisation.

Les cellules ont été placées dans la glace, lavées deux fois dans du PBS à la température de la glace et détachées de manière mécanique dans un tampon 1 (5 mM Tris, 2 mM EDTA, pH 7.4, 5 mg/litre d'inhibiteur de la trypsine de soja, 5 mg/litre de leupeptin, et 10 mg/litre de benzamidine) à la température de la glace.

Les suspensions cellulaires sont homogénéisées avec un homogénéiseur Polytron (Janke & Kunkel Ultra-Turrax T25) trois fois durant 5 sec. Le lysat est centrifugé à 450 X g durant 5 min à 4°C et le surnageant est centrifugé à 48000 X g durant 30 min à 4°C. Le culot final est lavé deux fois dans du tampon 1 et resuspendu dans une solution (75 mM Tris (pH 7.4), 12.5 mM MgCl₂, 5 mM EDTA avec des inhibiteurs de protéases, comme décrits ci dessus) et immédiatement utilisé dans des expériences de liaison de ligands radioactifs ou des expériences de BRET.

### Immunoprécipitation de JAK2

Des cellules HeLa ont été co-transfectées avec des plasmides exprimant JAK2 marquée par HA2 (don de Dr. Wojchowski, Pennsylvania State University, Pennsylvania, USA) et des plasmides contenant différentes constructions d'OBR. Les cellules ont été lysées dans du tampon de lyse (10 mM Tris, 150 mM NaCl, 5 mM EDTA, 5% glycerol, 0,02% NaN3, NP40 0,1%, orthovanadate 1mM, 5 mg/litre d'inhibiteur de la trypsine de soja et 10 mg/litre de benzamidine) et centrifugées durant 15 min à 13000 rpm. La fraction soluble a été immunoprécipitée durant 2h avec un anticorps polyclonal anti-JAK2 (HR-758) (1µg/ml) (Santa-Cruz Biotechnology, Santa Cruz, CA).

### Immmunoabsorption SDS-Page

Les immunoprécipitats JAK2 ont été dénaturés dans la solution (62.5 mM Tris/HCl (pH 6.8), 5% SDS, 10% glycerol et 0.05% bleu de bromophenol), à 100°C durant 10 minutes. Les protéines ont été séparées par SDS-PAGE en 7 % de poly-acrylamide et transferées sur nitrocellulose. L'immuno-détection a été effectuée avec un anticorps anti-phosphotyrosine 4G10 (2 µg/ml) (Upstate Biotechnology, Lake Placid, NY). L'immunoréactivité a été révélée en utilisant un anticorps secondaire approprié couplé à la peroxydase de raifort et le réactif chimio-luminescent ECL (Amersham, Aylesbury, UK).

### Test de liaison de la ¹²⁵I-leptine

Des cellules transfectées ont été carencées en sérum dans du DMEM (1% BSA) 24 h avant les expériences de liaison. Pour mesurer la liaison de la leptine à la surface des cellules, des cellules ont été lavées deux fois avec du PBS à la température de la glace et incubées dans un tampon de liaison (DMEM, 25 mM Hepes pH 7.4, 1% BSA) contenant 100000 cpm/puits de ¹²⁵I-leptine (PerkinElmer life sciences, Paris, France) en absence ou en présence de 200 nM de leptin non radioactive (leptine humaine recombinante (PeproTech Inc, USA) durant 4 h à 4°C. Les cellules ont été lavées deux fois avec du PBS à la température de la glace, lysées dans 1N NaOH et la radioactivité déterminée dans un compteur à radiations gammma. Afin de mesurer la quantité totale de leptine se liant dans l'extrait, les cellules ont été mises en suspension dans 1.5 ml de tampon de liaison contenant 0.15% de digitonine durant 2h à 4°C. Les extraits ont été centrifugés durant 30 min dans une centrifugeuse Eppendorf à vitesse maximale à 4°C. Le surnageant (0.2 ml) a été incubé avec 100000 cpm de ¹²⁵I-leptine en présence ou en absence de 200 nM de leptine dans un volume total de 0.25 ml avec agitation toute la nuit à 4°C.
0.5 ml de γ-globuline (1.25 mg/ml) et 0.5 ml de polyethylene glycol 6000 (25% p/v) ont été ajoutés pour précipiter les complexes récepteur-ligand , qui sont obtenus par centrifugation (17000 x g durant 3 min). Le culot est lavé avec 1 ml de polyéthylene glycol 6000 12 % (p/v) puis compté.

### Activation du gène rapporteur

Des cellules HeLa ont été co-transfectées avec 2.6 µg de plasmide portant le gène reporteur STAT3 (don de Dr. Levy, New York University, New York, USA), 200 pg de pcDNA3 comprenant la région codante de la Renilla luciferase (utilisée comme témoin interne) et avec 1.4 µg des différentes constructions OBR ou avec le véhicule seul. 48 heures après la transfection, les cellules ont été carencées durant la nuit en DMEM (1% BSA) avant la stimulation par 1nM de leptine durant 6 - 8 heures. Les cellules ont alors été lavées et lysées dans un tampon de lyse passive (Promega Corporation, Madison, WI) durant 15 minutes à température ambiante. Les lysats totaux ont été centrifugé durant 2 minutes à 15000 rpm et les surnageants ont été utilisés dans un test de dosage de la Luciferase ((Promega Corporation, Madison, WI) en utilisant un luminomètre Berthold (Lumat LB 9507). Les résultats sont exprimés par le rapport des activités luciférase de luciole ((firefly) / luciférase de *Renilla.*

### Mesure de BRET

48 heures après la transfection des cellules HeLa, COS et HEK 293 exprimant OBR ont été détachées et lavées avec du PBS. 1-2x10⁵ cellules ont été distribuées dans des plaques optiques à 96 puits (Packard Instrument Company, Meriden, CT) en absence ou en présence de ligands, à 25°C. Des membranes préparées à partir de cellules exprimant OBR ont été utilisées pour les mesures de BRET. Le substrat, la coelenterazine h (Molecular Probes, Eugene, OR) est ajouté à une concentration finale de 5 µM et la lecture est faite avec un fluoro/luminométre Fusion^{™} (Packard Instrument Company, Meriden, CT) qui permet l'intégration séquentielle des signaux de luminescence détectés avec deux filtres (filtre Luc: 485 ± 10 nm; filtre YFP: 530 ± 12.5 nm). Le rapport BRET est défini comme la différence de l'émission à 530 nm/485 nm des protéine de fusion Luc et YFP co-transfectées et l'émission à 530 nm/485 nm de la protéine de fusion Luc seule. Les résultats sont exprimés en unités milliBRET (mBU), 1 unité mBRET correspondant à la valeur du rapport BRET multiplié par 1000. Les ligands suivants ont été utilisés pour déterminer la spécificité du dosage: érythropoiétine humaine recombinante (EPO), insuline (Ins), lipopolysaccharide (LPS, Sigma Aldrich, St Louis, USA), trombopoiétine Humaine recombinante (TPO), GM-CSF, interleukine 3 (IL3), interleukine 6 (IL6), prolactine (PRL), SCF, EGF et TNFα.

### Exemple 1 : Expression fonctionnelle des protéines de fusion OBR

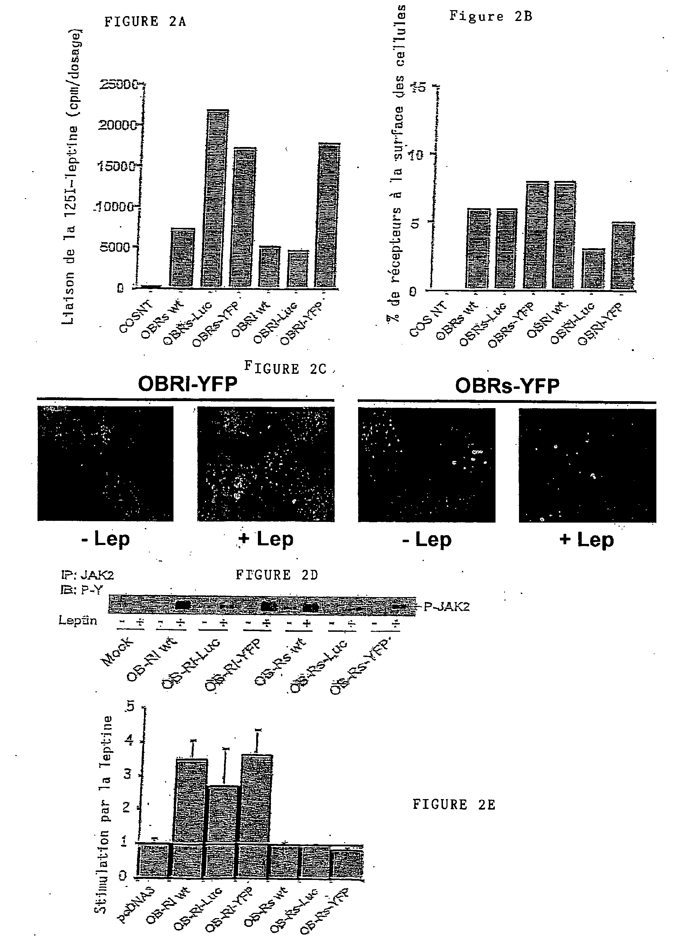

La forme longue (OBR1) et la forme courte (OBRs) des OBR on été fusionnées à leurs extrémités C-terminale avec la YFP ou la Luc (Fig. 1). L'expression de ces protéines de fusion a été confirmée dans des cellules COS transfectées dans des expériences de liaison avec la ¹²⁵I-leptin (Fig. 2a). Des résultats similaires ont été obtenus dans des cellules HeLa transfectées.

L'expression à la surface des cellules des protéines de fusion et des récepteurs de type sauvage exprimés dans les cellules COS varient entre 5 et 10 %, ce qui est en accord avec des valeurs déjà décrites. Des valeurs similaires sont obtenues dans des cellules HEK 293 exprimant des OBR endogènes (14 ± 3 %).
La localisation des protéines de fusion OBR dans les cellules HeLa a été étudiée par microscopie de fluorescence en utilisant les protéines de fusion avec YFP. La fluorescence due à OBR1-YFP est repartie ponctuellement dans les cellules tandis que celle due à OBRs-YFP est localisée dans des plaques. La stimulation par la leptine localise OBR1-YFP dans de grandes plaques intracellulaires correspondant probablement au compartiment endosomal. La localisation de OBRs-YFP ne change pas de manière significative. Les résultats obtenus par microscopie à fluorescence confirment la localisation prédominante de OBR dans le compartiment intracellulaire et sont cohérents avec la localisation déjà décrite de la protéine de fusion OBR1-GFP dans des cellules COS.
L'expression fonctionnelle des protéines de fusion est évaluée par mesure de l'activation de la voie JAK-STAT. Les kinases JAK2 sont associées avec les domaines intracellulaires de OBRs et OBR1. La liaison de ligands induit la transphosphorylation de JAK2 et la phosphorylation de OBR1 mais pas d'OBRs. OBRl phosphorylé fournit ensuite un site d'accrochage pour les protéines STAT, qui sont activées par phosphorylation de la tyrosine après liaison au récepteur. Les protéines STAT activées dimèrisent alors et sont transloquées au noyau où elles stimulent la transcription de gènes par l'intermédiaire d'éléments de réponse au STAT comme décrit par Tartaglia (1997, J Biol Chem 272, 6093-6096).
Comme le montre la figure 2c toutes les constructions OBRs induisent la phosphorylation de JAK2 ce qui indique une activation de JAK2. L'activité du gène rapporteur STAT3 est activée d'un facteur 2-4 par OBRI-wt et les protéines de fusion OBR1 tandis que les isoformes courtes n'ont pas d'effet sur l'activité du gène rapporteur. Ces résultats indiquent que les protéines de fusion OBR sont fonctionnellement exprimées dans les cellules HeLa.

### Exemple 2: Dimerisation constitutive de OBR dans des cellules vivantes.

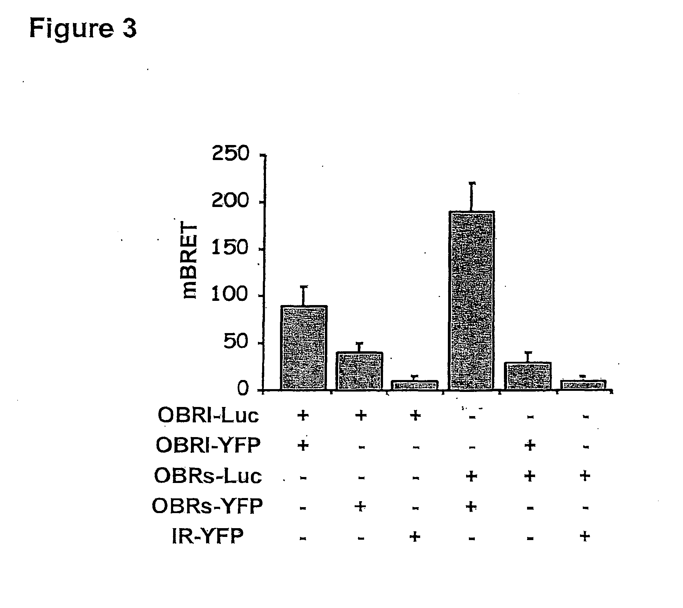

La dimèrisation de OBR-Luc et OBR-YFP a été étudiée dans des cellules vivantes.
Des transferts d'énergie significatifs ont été observés entre OBRs-Luc et OBRs-YFP ainsi qu'entre OBRI-Luc et OBRI-YFP, exprimés dans des quantités équimolaires, ce qui indique que des homo-dimères constitutifs existent pour les deux récepteurs (Fig. 3 a, b). L'existence des hétéro-dimères OBRs/OBR1 dans les cellules vivantes est démontrée par la détection de BRET entre OBRs-Luc et OBR1-YFP ainsi qu'entre OBRI-Luc et OBRs-YFP. La spécificité de ces interactions est illustrée par l'absence de transfert significatif entre OBRs-Luc et OBRI-Luc et une protéine fusion entre l'YFP et le récepteur de l'insuline décrite récemment (Boute et al., 2001 précédemment cités).
Ces résultats indiquent que les isoformes courtes et longues sont impliqués dans des hétéro et homo complexes dans les cellules vivantes.

### Exemple 3: Effet de la liaison de la leptine sur le BRET constitutif des OBR :

Afin d'évaluer les effets agonistes sur le BRET constitutif, les cellules ont été pré-incubées avec la leptine avant d'initier la réaction de la luciférase avec son substrat. Aucun changement dans le BRET constitutif n'est observé avec les homodimères OBR1 et les deux combinaisons d'hétéro dimères OBRs/OBR1 alors que le

BRET est augmenté avec les homo dimères OBRs (Fig. 4 a). Les changements de BRET des homo dimères OBRs induits par la leptine ont ensuite été mesurés dans différentes préparations cellulaires.
La rupture mécanique des cellules dans un tampon hypotonique améliore de manière significative l'augmentation du BRET par la leptine tandis que le BRET basal reste inchangé.
Des résultats similaires ont été obtenus avec la fraction membranaire après séparation du cytosol. Tandis que tous les couples OBRs-Luc/OBRs-YFP contribuent au BRET basal, seuls les récepteurs exposés à la surface de la cellule (5-10 %) peuvent être stimulés par la leptine qui est imperméable aux membranes dans des cellules intactes. La disruption des membranes cellulaires augmente la fraction d'OBR accessible à la leptine et qui est responsable de l'augmentation du BRET induit par la leptine.

Des résultats similaires ont été obtenus sur cellules traitées avec la saponine. Ce composant fait des trous dans les membranes et permet la pénétration des protéines comme la leptine dans les compartiments intracellulaires ou se trouve la majorité des OBRs.
Aucun changement de BRET induit par la leptine n'a été observé dans des expériences analogues effectuées avec des préparations à partir de cellules exprimant des homo-dimères OBR1 -ou des hétéro-dimères OBRs/OBR1.
Les quantités de et les rapports de OBRs-Luc et OBRs-YFP ont ensuite été modulés afin d'optimiser le BRET induit par la leptine (Fig. 5a). Les meilleurs résultats sont obtenus quand 500 ng d'ADN codant pour OBRs-Luc et 250 ng d'ADN codant pour OBRs-YFP sont utilisés.
Dans ces conditions optimisées une concentration saturée de leptine induit une augmentation d'un facteur 2 - 2.5 du signal BRET basal dans des cellules incubées avec la saponine ou membranes préparées à partir de cellules exprimant des homodimères OBRs. Cette augmentation est fonction du temps. Les valeurs maximales sont atteintes après 20 minutes d'incubation avec 1nM leptine à température ambiante(Fig. 5b). Pour des concentrations supérieures en leptine, les valeurs maximales sont obtenues après 5 minutes d'incubation à température ambiante.
L'effet de la leptine est dose-dépendant avec un EC50 d'environ 100 pM (Fig. 5c), ce qui est en accord avec les valeurs de Ki obtenues avec les protéines de fusion OBRs-Luc (116 pM) et OBRsYFP (35 pM) (Fig 5 d). La spécificité du test est démontrée par l'absence de BRET induite par le ligand par une concentration saturante de plusieurs cytokines et d'autres ligands de récepteurs membranaires tels que l'érythropoiétine, la trombopoiétine, le GM-CSF, l'IL3, l'IL6, la PRL, le SCFα, l'EGF, l'insuline, le LPS et le TNFα.
La répartition des récepteurs en dimères suit des lois statistiques, et à un rapport 1/1 en nombre de récepteurs on s'attend à la répartition suivante si tous les récepteurs sont sous forme dimérique: 1/4 Luc/Luc, 1/4 YFP/YFP et 1/2 de récepteurs capables d'engendrer un signal de BRET (1/4 Luc/YFP, 1/4 YFP/Luc). Cependant lors des mesures de BRET, l'ensemble des molécules fusionnées à la Luc donnent un signal de luminescence et donc à un rapport 1/1 on observe la moitié des récepteurs capables de BRET, sur une population donneuse totale. Aussi, pour augmenter le signal de BRET, on a réalisé des expériences de saturation des molécules Luc par les molécules YFP de façon à avoir l'ensemble des molécules Luc sous forme de dimères avec les molécules YFP (capables de BRET). Les résultats de la figure 6, montrent que le signal de BRET basal augmente lors de la saturation et que l'induction par la leptine est proportionnelle au signal basal, avec une stimulation de 2-2,5 fois le BRET basal. A saturation on obtient une meilleure résolution du BRET basal et induit, permettant un criblage plus aisé pour la recherche de molécules.

## Revendications

1. Procédé de détermination de la liaison de composés au récepteur de la leptine comprenant les étapes consistant à :
a) -mettre en contact lesdits composés avec une protéine de fusion composée d'un récepteur de la leptine présentant un domaine intracellulaire court ou d'une forme soluble d'un tel récepteur contenant le site de liaison à la leptine, et d'une protéine donneur d'énergie et une protéine de fusion composée d'un récepteur de la leptine présentant un domaine intracellulaire court ou d'une forme soluble d'un tel récepteur contenant le site de liaison à la leptine et d'une protéine accepteur d'énergie ou à mettre en contact lesdits composés avec des cellules, ou des fragments, ou des lysats, ou des membranes de cellules comprenant de telles protéines de fusion, et éventuellement un substrat enzymatique adéquat, et
b) -à mesurer le transfert d'énergie.

2. Procédé selon la revendication 1 **caractérisé en ce que** le transfert d'énergie mesuré en présence du composé à tester est comparé à celui mesuré en absence du composé à tester.

3. Procédé de criblage ou de détection de composés destinés à la prévention et / ou au traitement de pathologies comprenant les étapes consistant à :
a) -mettre en contact lesdits composés avec une protéine de fusion composée d'un récepteur de la leptine présentant un domaine intracellulaire court ou d'une forme soluble d'un tel récepteur contenant le site de liaison à la leptine et d'une protéine donneur d'énergie et une protéine de fusion composée d'un récepteur de la leptine présentant un domaine intracellulaire court ou d'une forme soluble d'un tel récepteur contenant le site de liaison à la leptine et d'une protéine accepteur d'énergie, ou des cellules, ou des fragments, ou des lysats, ou des membranes de cellules comprenant de telles protéines, et éventuellement un substrat enzymatique adéquat, et
b)-à mesurer le transfert d'énergie.

4. Procédé de criblage d'agonistes ou d'antagonistes du récepteur de la leptine comprenant les étapes consistant à :
a)-mettre en contact les agonistes ou antagonistes potentiels avec une protéine de fusion composée d'un récepteur de la leptine présentant un domaine intracellulaire court ou d'une forme soluble d'un tel récepteur contenant le site de liaison à la leptine et d'une protéine donneur d'énergie et une protéine de fusion composée d'un récepteur de la leptine présentant un domaine intracellulaire court ou d'une forme soluble d'un tel récepteur contenant le site de liaison à la leptine et d'une protéine accepteur d'énergie ou à mettre en contact lesdits composés avec des cellules, ou des fragments, ou des lysats, ou des membranes de cellules comprenant de telles protéines de fusion, et éventuellement un substrat enzymatique adéquat, et
b)-à mesurer le transfert d'énergie.

5. Procédé selon l'une des revendications 1 à 4 **caractérisé en ce que** le récepteur de la leptine est une isoforme courte.

6. Procédé selon l'une des revendications 1 à 5 **caractérisé en ce que** le récepteur de la leptine est une isoforme comprenant un domaine intracellulaire Box1, mais ne comprenant pas de domaine intracellulaire Box 3.

7. Procédé selon l'une des revendications 1 à 6 **caractérisé en ce que** le récepteur de la leptine est l'isoforme OBRs.

8. Procédé selon l'une des revendications 1 à 4 **caractérisé en ce que** le récepteur de la leptine est l'isoforme OBRs humaine de séquence SEQ ID N°2, ou un variant de ce récepteur présentant une identité d'au moins 65% avec la séquence SEQ ID N°2

9. Procédé selon l'une des revendications 1 à 4 **caractérisé en ce que** la séquence du récepteur de la leptine est la séquence des acides aminés 46 à 866 de l'isoforme OBRs humaine de séquence SEQ ID N°2, ou un variant de cette séquence présentant une identité d'au moins 65%.

10. Procédé selon l'une des revendications 1 à 4 **caractérisé en ce que** le récepteur de la leptine présente la séquence SEQ ID N°4, ou un variant de cette séquence présentant une identité d'au moins 65%.

11. Procédé selon l'une des revendications 1 à 10 **caractérisé en ce que** la protéine de fusion donneur d'énergie est une protéine de fusion entre le récepteur de la leptine et la luciférase.

12. Procédé selon l'une des revendications 1 à 11 **caractérisé en ce que** la protéine de fusion accepteur d'énergie est une protéine de fusion entre le récepteur de la leptine et la GFP ou un mutant de cette protéine ou la DsRed.

13. Procédé selon la revendication 12 **caractérisé en ce que** le mutant de la GFP est la YFP, l'EYFP, la GFPS65T, ou la Topaz.

14. Procédé selon l'une des revendications 1 à 10 **caractérisé en ce que** la protéine de fusion donneur d'énergie présente la séquence SEQ ID N°6 ou un variant de cette séquence présentant une identité d'au moins 65%.

15. Procédé selon l'une des revendications 1 à 10 **caractérisé en ce que** la protéine de fusion accepteur d'énergie présente la séquence SEQ ID N°8 ou un variant de cette séquence présentant une identité d'au moins 65%.

16. Procédé selon l'une des revendications 1 à 15 **caractérisé en ce qu'**il est mis en oeuvre avec les cellules traitées avec de la saponine.

17. Procédé selon l'une des revendications 1 à 16 **caractérisé en ce que** le substrat est la coelenterazine.

## Claims

1. Method for determining the binding of compounds to the leptin receptor, comprising the steps consisting in:
a) - bringing said compounds into contact with a fusion protein composed of a leptin receptor having a short intracellular domain or of a soluble form of such a receptor containing the leptin-binding site, and of an energy donor protein, and with a fusion protein composed of a leptin receptor having a short intracellular domain or of a soluble form of such a receptor containing the leptin-binding site, and of an energy acceptor protein, or bringing said compounds into contact with cells, or fragments or lysates or membranes of cells comprising such proteins, and optionally an appropriate enzyme substrate, and
b) - measuring the energy transfer.

2. Method according to Claim 1, **characterized in that** the energy transfer measured in the presence of the test compound is compared to that measured in the absence of the test compound.

3. Method for screening or detecting compounds intended for the prevention and/or treatment of pathological conditions, comprising the steps consisting in:
a) - bringing said compounds into contact with a fusion protein composed of a leptin receptor having a short intracellular domain or of a soluble form of such a receptor containing the leptin-binding site, and of an energy donor protein, and with a fusion protein composed of a leptin receptor having a short intracellular domain or of a soluble form of such a receptor containing the leptin-binding site, and of an energy acceptor protein, or bringing said compounds into contact with cells, or fragments or lysates or membranes of cells comprising such proteins, and optionally an appropriate enzyme substrate, and
b) - measuring the energy transfer.

4. Method for screening leptin receptor agonists or antagonists, comprising the steps consisting in:
a) - bringing the potential agonists or antagonists into contact with a fusion protein composed of a leptin receptor having a short intracellular domain or of a soluble form of such a receptor containing the leptin-binding site, and of an energy donor protein, and with a fusion protein composed of a leptin receptor having a short intracellular domain or of a soluble form of such a receptor containing the leptin-binding site, and of an energy acceptor protein, or bringing said compounds into contact with cells, or fragments or lysates or membranes of cells comprising such fusion proteins, and optionally an appropriate enzyme substrate, and
b) - measuring the energy transfer.

5. Method according to one of Claims 1 to 4, **characterized in that** the leptin receptor is a short isoform.

6. Method according to one of Claims 1 to 5, **characterized in that** the leptin receptor is an isoform comprising a Box1 intracellular domain, but not comprising a Box 3 intracellular domain.

7. Method according to one of Claims 1 to 6, **characterized in that** the leptin receptor is the OBRs isoform.

8. Method according to one of Claims 1 to 4, **characterized in that** the leptin receptor is the human OBRs isoform of sequence SEQ ID No. 2, or a variant of this receptor exhibiting at least 65% identity with the sequence SEQ ID No. 2.

9. Method according to one of Claims 1 to 4, **characterized in that** the sequence of the leptin receptor is the sequence of amino acids 46 to 866 of the human OBRs isoform of sequence SEQ ID No. 2, or a variant of this sequence exhibiting at least 65% identity.

10. Method according to one of Claims 1 to 4, **characterized in that** the leptin receptor has the sequence SEQ ID No. 4, or a variant of this sequence exhibiting at least 65% identity.

11. Method according to one of Claims 1 to 10, **characterized in that** the energy donor fusion protein is a protein from fusion between the leptin receptor and luciferase.

12. Method according to one of Claims 1 to 11, **characterized in that** the energy acceptor fusion protein is a protein from fusion between the leptin receptor and GFP or a mutant of this protein or DsRed.

13. Method according of Claim 12, **characterized in that** the GFP mutant is YFP, EYFP, GFPS65T, or Topaz.

14. Method according to one of Claims 1 to 10, **characterized in that** the energy donor fusion protein has the sequence SEQ ID No. 6, or a variant of this sequence exhibiting at least 65% identity.

15. Method according to one of Claims 1 to 10, **characterized in that** the energy donor fusion protein has the sequence SEQ ID No. 8, or a variant of this sequence exhibiting at least 65% identity.

16. Method according to one of Claims 1 to 15, **characterized in that** it is used with the cells treated with saponin.

17. Method according to one of Claims 1 to 16, **characterized in that** the substrate is coelenterazine.

## Patentansprüche

1. Verfahren zur Ermittlung der Bindung von Verbindungen an den Leptinrezeptor, das die Schritte umfasst, die daraus bestehen:
a) besagte Verbindungen in Kontakt zu bringen mit einem Fusionsprotein, das aus einem Leptinrezeptor, der eine kurze intrazelluläre Domäne aufweist, oder aus einer löslichen Form eines solchen Rezeptors, die die Stelle der Bindung an das Leptin enthält, und aus einem Energiedonor-Protein zusammengesetzt ist, und einem Fusionsprotein, das aus einem Leptinrezeptor, der eine kurze intrazelluläre Domäne aufweist, oder aus einer löslichen Form eines solchen Rezeptors, die die Stelle der Bindung an das Leptin enthält, und aus einem Energieakzeptor-Protein zusammengesetzt ist, oder besagte Verbindungen mit Zellen oder Fragmenten oder Lysaten oder Zellmembranen, die solche Fusionsproteine umfassen, und gegebenenfalls einem geeigneten Enzymsubstrat in Kontakt zu bringen und
b) den Energietransfer zu messen.

2. Verfahren gemäß Anspruch 1, **dadurch gekennzeichnet, dass** der in Gegenwart der zu untersuchenden Verbindung gemessene Energietransfer mit dem in Abwesenheit der zu untersuchenden Verbindung gemessenen verglichen wird.

3. Verfahren zum Screening oder zum Nachweis von Verbindungen, die für die Prophylaxe und/oder die Behandlung von Erkrankungen bestimmt sind, das die Schritte umfasst, die daraus bestehen:
a) besagte Verbindungen in Kontakt zu bringen mit einem Fusionsprotein, das aus einem Leptinrezeptor, der eine kurze intrazelluläre Domäne aufweist, oder aus einer löslichen Form eines solchen Rezeptors, die die Stelle der Bindung an das Leptin enthält, und aus einem Energiedonor-Protein zusammengesetzt ist, und einem Fusionsprotein, das aus einem Leptinrezeptor, der eine kurze intrazelluläre Domäne aufweist, oder aus einer löslichen Form eines solchen Rezeptors, die die Stelle der Bindung an das Leptin enthält, und aus einem Energieakzeptor-Protein zusammengesetzt ist, oder mit Zellen oder Fragmenten oder Lysaten oder Zellmembranen, die solche Proteine umfassen, und gegebenenfalls einem geeigneten Enzymsubstrat und
b) den Energietransfer zu messen.

4. Verfahren zum Screening von Agonisten oder Antagonisten des Leptinrezeptors, das die Schritte umfasst, die daraus bestehen:
a) die möglichen Agonisten oder Antagonisten in Kontakt zu bringen mit einem Fusionsprotein, das aus einem Leptinrezeptor, der eine kurze intrazelluläre Domäne aufweist, oder aus einer löslichen Form eines solchen Rezeptors, die die Stelle der Bindung an das Leptin enthält, und aus einem Energiedonor-Protein zusammengesetzt ist, und einem Fusionsprotein, das aus einem Leptinrezeptor, der eine kurze intrazelluläre Domäne aufweist, oder aus einer löslichen Form eines solchen Rezeptors, die die Stelle der Bindung an das Leptin enthält, und aus einem Energieakzeptor-Protein zusammengesetzt ist, oder besagte Verbindungen mit Zellen oder Fragmenten oder Lysaten oder Zellmembranen, die solche Fusionsproteine umfassen, und gegebenenfalls einem geeigneten Enzymsubstrat in Kontakt zu bringen und
b) den Energietransfer zu messen.

5. Verfahren gemäß einem der Ansprüche 1 bis 4, **dadurch gekennzeichnet, dass** der Leptinrezeptor eine kurze Isoform ist.

6. Verfahren gemäß einem der Ansprüche 1 bis 5, **dadurch gekennzeichnet, dass** der Leptinrezeptor eine Isoform ist, die eine intrazelluläre Domäne Box 1 umfasst, aber keine intrazelluläre Domäne Box 3 umfasst.

7. Verfahren gemäß einem der Ansprüche 1 bis 6, **dadurch gekennzeichnet, dass** der Leptinrezeptor die Isoform OBRs ist.

8. Verfahren gemäß einem der Ansprüche 1 bis 4, **dadurch gekennzeichnet, dass** der Leptinrezeptor die humane Isoform OBRs mit der Sequenz SEQ ID NO: 2 oder eine Variante dieses Rezeptors ist, die zu wenigstens 65 % mit der Sequenz SEQ ID NO: 2 identisch ist.

9. Verfahren gemäß einem der Ansprüche 1 bis 4, **dadurch gekennzeichnet, dass** die Sequenz des Leptinrezeptors die Sequenz der Aminosäuren 48 bis 866 der humanen Isoform OBRs mit der Sequenz SEQ ID NO: 2 oder eine Variante dieser Sequenz ist, die zu wenigstens 65 % identisch ist.

10. Verfahren gemäß einem der Ansprüche 1 bis 4, **dadurch gekennzeichnet, dass** der Leptinrezeptor die Sequenz SEQ ID NO: 4 oder eine Variante dieser Sequenz, die zu wenigstens 65 % identisch ist, aufweist.

11. Verfahren gemäß einem der Ansprüche 1 bis 10, **dadurch gekennzeichnet, dass** das Energiedonor-Fusionsprotein ein Fusionsprotein zwischen dem Leptinrezeptor und Luziferase ist.

12. Verfahren gemäß einem der Ansprüche 1 bis 11, **dadurch gekennzeichnet, dass** das Energieakzeptor-Fusionsprotein ein Fusionsprotein zwischen dem Leptinrezeptor und GFP oder einer Mutante dieses Proteins oder DsRed ist.

13. Verfahren gemäß Anspruch 12, **dadurch gekennzeichnet, dass** die Mutante des GFP YFP, EYFP, GFPS65T oder Topaz ist.

14. Verfahren gemäß einem der Ansprüche 1 bis 10, **dadurch gekennzeichnet, dass** das Energiedonor-Fusionsprotein die Sequenz SEQ ID NO: 6 oder eine Variante dieser Sequenz, die zu wenigstens 65 % identisch ist, aufweist.

15. Verfahren gemäß einem der Ansprüche 1 bis 10, **dadurch gekennzeichnet, dass** das Energieakzeptor-Fusionsprotein die Sequenz SEQ ID NO: 8 oder eine Variante dieser Sequenz, die zu wenigstens 65 % identisch ist, aufweist.

16. Verfahren gemäß einem der Ansprüche 1 bis 15, **dadurch gekennzeichnet, dass** es mit Zellen, die mit Saponin behandelt sind, durchgeführt wird.

17. Verfahren gemäß einem der Ansprüche 1 bis 16, **dadurch gekennzeichnet, dass** das Substrat Coelenterazin ist.
